# EUROPÄISCHE PATENTSCHRIFT

(11) **EP 0 810 206 B1**
(45) Veröffentlichungstag und Bekanntmachung des Hinweises auf die Patenterteilung: **27.12.2000**
(21) Anmeldenummer: 97108013.0
(22) Anmeldetag: 16.05.1997
(51) Int. Cl.: C07C 271/22, A61K 31/155, C07D 295/08, C07D 213/40

(54) **Substituierte 2-Naphthoylguanidine, Verfahren zu ihrer Herstellung, ihre Verwendung als Medikament oder Diagnostikum sowie sie enthaltendes Medikament**
Substituted 2-naphthoylguanidines, process for their preparation, their use as medicament or diagnostic agent and medicament containing them
Naphtoylguanides 2-substituées, procédé pour leur préparation, leur utilisation comme médicament ou agent de diagnostique et médicaments les contenant

(30) Priorität: 29.05.1996 DE 19621483
(43) Veröffentlichungstag der Anmeldung: 03.12.1997
(73) Patentinhaber: HOECHST AKTIENGESELLSCHAFT, 65929 Frankfurt am Main (DE)
(72) Erfinder: Brendel, Joachim, Dr., 61118 Bad Vilbel (DE); Kleemann, Heinz-Werner, Dr., 65474 Bischofsheim (DE); Englert, Heinrich Christian, Dr., 65719 Hofheim (DE); Lang, Hans Jochen, Dr., 65719 Hofheim (DE); Schwark, Jan-Robert, Dr., 65929 Frankfurt (DE); Weichert, Andreas, Dr., 63329 Egelsbach (DE); Lal, Bansi, Dr., Mulund (W) Bombay Pin 400 080 (IN)

(56) Entgegenhaltungen:
- EP-A- 0 483 667
- EP-A- 0 682 017
- WO-A-94/26709
- US-A- 4 251 545
- PATENT ABSTRACTS OF JAPAN vol. 097, no. 001, 31.Januar 1997 & JP 08 225513 A (KANEBO LTD), 3.September 1996,

## Beschreibung

Die Erfindung betrifft substituierte 2-Naphthoylguanidine der Formel I 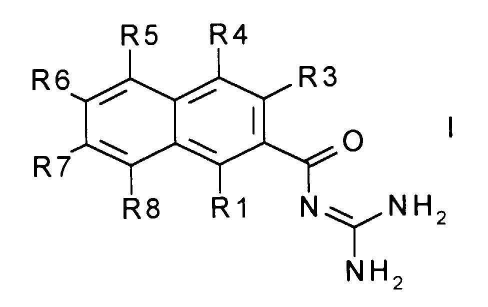 worin bedeuten:
mindestens einer der Substituenten R1, R3, R4, R5, R6, R7 und R8
XYₐ WZ oder X'YₐWZ';
X O, S, NR(10) oder CR(11)R(12);
R(10), R(11) und R(12)
unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
Y Alkylen mit 1, 2, 3, 4, 5, 6, 7 oder 8 CH₂-Gruppen,
wobei eine dieser CH₂-Gruppen durch 0, S, NR(13) oder o-, p- oder m-Phenylen ersetzt sein kann;
R(13) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;
a Null oder 1;
W CH₂, SO₂, oder - falls W nicht unmittelbar auf ein Heteroatom der Gruppe XYₐ folgt - auch O oder NR(14);
R(14) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;
Z C(=O)R(15), SO₂R(15) oder - falls W nicht O oder NR(14) bedeutet - auch NR(16)R(17);
R(15)
N=C(NH₂)₂, NR(18)R(19), N(CH₂)_{b}NR(18)R(19) oder OR(20);
b 2 oder 3;
R(18) und R(19)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(18) und R(19)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
R(20)
H, Alkyl mit 1, 2, 3, 4, 5 oder 6 C-Atomen,
Perfluoralkyl mit 1, 2, 3 oder 4 C-Atomen oder
Cycloalkyl mit 3, 4, 5, 6 oder 7 C-Atomen;
R(16) und R(17)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
X' C=O, C(=O)NR(30), C(=O)O, SO, SO₂, SO₂NR(30), OC=O, NR(30)C=O oder NR(30)SO₂,
wobei die Verknüpfung mit dem Naphthalinring jeweils über das links stehende Atom erfolgt;
R(30) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
Z' C(=O)R(15), SO₂R(15), einen N-haltigen Heterocyclus mit 1, 2, 3, 4, 5, 6, 7, 8 oder 9 C-Atomen,
wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(21)R(22);
R(21) und R(22)
unabhängig voneinander H, Alkyl mit 1, 2, 3 oder 4 C-Atomen oder Perfluoralkyl mit 1, 2, 3 oder 4 C-Atomen;
R(15)
N=C(NH₂)₂, NR(18)R(19), N(CH₂)_{b}NR(18)R(19) oder OR(20);
R(18) und R(19)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(18) und R(19)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
b 2 oder 3;
R(20) H, Alkyl mit 1, 2, 3, 4, 5 oder 6 C-Atomen,
Perfluoralkyl mit 1, 2, 3 oder 4 C-Atomen oder
Cycloalkyl mit 3, 4, 5, 6 oder 7 C-Atomen;
oder
Z' - falls W nicht O oder NR(14) bedeutet - NR(16)R(17);
R(16) und R(17)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann; und die jeweils übrigen Substituenten R1, R3, R4, R5, R6, R7 und R8, die noch nicht durch die zuvor gegebenen Definitionen vergeben sind,
unabhängig voneinander H, F, Cl, Br, I, CN, NO₂, CF₃, C₂F₅ oder VₚQ_{q}U;
V 0, S, SO, SO₂, NR(60), OC=O, C=O, C(=O)NR(60), C(=O)O oder CR(66)R(67);
R(60), R(66) und R(67)
unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
p Null oder 1;
Q Alkylen mit 1, 2, 3, 4, 5, 6, 7 oder 8 CH₂-Gruppen,
wobei eine dieser CH₂-Gruppen durch O, S, NR(68) oder o-, p- oder m-Phenylen ersetzt sein kann;
R(68)
H, Alkyl mit 1, 2, 3, 4, 5 oder 6 C-Atomen,
Perfluoralkyl mit 1, 2, 3 oder 4 C-Atomen oder
Cycloalkyl mit 3, 4, 5 oder 6 C-Atomen;
q Null oder 1;
U H, Alkyl mit 1, 2, 3, 4, 5, 6 oder 7 C-Atomen, Cycloalkyl mit 3, 4, 5, 6 oder 7 C-Atomen, C(=O)R(65), SO₂R(65), NR(61)R(62) oder Phenyl,
das nicht substituiert oder substituiert ist mit 1 - 3 Substituenten ausgewählt aus der Gruppe bestehend aus F, Cl, Br, CF₃, Methyl, Methoxy und NR(63)R(64);
R(63) und R(64)
unabhängig voneinander H, Alkyl mit 1, 2, 3 oder 4 C-Atomen oder Perfluoralkyl mit 1, 2, 3 oder 4 C-Atomen; R(65) N=C(NH₂)₂ , NR(61)R(62) oder OR(60);
R(61) und R(62)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(61) und R(62)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
oder
U einen N-haltigen Heterocyclus mit 1, 2, 3, 4, 5, 6, 7, 8 oder 9 C-Atomen,
wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(63)R(64); wobei jedoch mindestens einer der Substituenten R5, R6, R7 und R8 ungleich Wasserstoff ist;
sowie deren pharmazeutisch verträgliche Salze.

Bevorzugt sind Verbindungen der Formel I, in denen bedeuten:
mindestens einer der Substituenten R1, R3, R4, R5, R6, R7 und R8
XYₐ WZ oder X'YₐWZ';
X O, S, NR(10);
R(10) H, Alkyl mit 1, 2, 3 oder 4 C-Atomen, Perfluoralkyl mit 1 oder 2 C-Atomen oder Cycloalkyl mit 3, 4, 5 oder 6 C-Atomen;
Y Alkylen mit 1, 2, 3, 4 oder 5 CH₂-Gruppen,
wobei eine dieser CH₂-Gruppen durch O, S, NR(13) oder o-, p- oder m-Phenylen ersetzt sein kann;
R(13) H, Alkyl mit 1, 2, 3 oder 4 C-Atomen oder Cycloalkyl mit 3, 4, 5 oder 6 C-Atomen;
a Null oder 1;
W CH₂ oder - falls W nicht unmittelbar auf ein Heteroatom der Gruppe XYₐ folgt - auch O oder NR(14);
R(14) H, Alkyl mit 1, 2, 3 oder 4 C-Atomen;
Z C(=O)R(15), SO₂R(15) oder - falls W nicht O oder NR(14) bedeutet - auch NR(16)R(17);
R(15) N=C(NH₂)₂, NR(18)R(19), N(CH₂)_{b}NR(18)R(19) oder OR(20);
b 2 oder 3;
R(18) und R(19)
unabhängig voneinander H oder Alkyl mit 1, 2, 3, 4 oder 5 C-Atomen;
oder
R(18) und R(19)
gemeinsam 4 oder 5 Methylengruppen,
von denen eine CH₂-Gruppe durch Sauerstoff, S, NH, N-CH₃ oder N-Benzyl ersetzt sein kann;
R(20) H, Alkyl mit 1, 2, 3, 4, 5 oder 6 C-Atomen;
R(16) und R(17)
unabhängig voneinander H, Alkyl mit 1, 2, 3, 4 oder 5 C-Atomen oder Perfluoralkyl mit 1 oder 2 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
X' C(=O)NR(30), C(=O)O, SO₂NR(30), OC=O, NR(30)C=O oder NR(30)SO₂,
wobei die Verknüpfung mit dem Naphthalinring jeweils über das links stehende Atom erfolgt;
R(30) H, Alkyl mit 1, 2, 3 oder 4 C-Atomen, Perfluoralkyl mit 1 oder 2 C-Atomen oder Cycloalkyl mit 3, 4, 5 oder 6 C-Atomen;
Z' C(=O)R(15), SO₂R(15), einen N-haltigen Heterocyclus mit 1, 2, 3, 4, 5, 6, 7, 8 oder 9 C-Atomen,
wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(21)R(22);
R(21) und R(22)
unabhängig voneinander H, Alkyl mit 1, 2, 3 oder 4 C-Atomen;
R(15) N=C(NH₂)₂, NR(18)R(19), N(CH₂)_{b}NR(18)R(19) oder OR(20);
R(18) und R(19)
unabhängig voneinander H oder Alkyl mit 1, 2, 3, 4 oder 5 C-Atomen;
oder
R(18) und R(19)
gemeinsam 4 oder 5 Methylengruppen,
von denen eine CH₂-Gruppe durch Sauerstoff, S, NH, N-CH₃ oder N-Benzyl ersetzt sein kann;
b 2 oder 3;
R(20) H, Alkyl mit 1, 2, 3, 4, 5 oder 6 C-Atomen;
oder
Z' - falls W nicht O oder NR(14) bedeutet - NR(16)R(17);
R(16) und R(17)
unabhängig voneinander H, Alkyl mit 1, 2, 3, 4 oder 5 C-Atomen oder Perfluoralkyl mit 1 oder 2 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
und die jeweils übrigen Substituenten R1, R3, R4, R5, R6, R7 und R8, die noch nicht durch die zuvor gegebenen Definitionen vergeben sind,
unabhängig voneinander H, F, Cl, Br, I, CN, NO₂, CF₃, C₂F₅ oder VₚQ_{q}U;
V O, S, SO₂, NR(60), OC=O, C=O, C(=O)NR(60), C(=O)O oder CR(66)R(67);
R(60), R(66) und R(67)
unabhängig voneinander H, Alkyl mit 1, 2 oder 3 C-Atomen, Perfluoralkyl mit 1 oder 2 C-Atomen oder Cycloalkyl mit 3, 4, 5 oder 6 C-Atomen;
p Null oder 1;
q Null;
U H, Alkyl mit 1, 2, 3 oder 4 C-Atomen, Cycloalkyl mit 3, 4, 5 oder 6 C-Atomen, C(=O)R(65), NR(61)R(62) oder Phenyl,
das nicht substituiert oder substituiert ist mit 1 - 3 Substituenten ausgewählt aus der Gruppe bestehend aus F, Cl, Br, CF₃, Methyl, Methoxy und NR(63)R(64);
R(63) und R(64)
unabhängig voneinander H, Alkyl mit 1, 2, 3 oder 4 C-Atomen;
R(65) N=C(NH₂)₂ oder OR(60);
R(61) und R(62)
unabhängig voneinander H, Alkyl mit 1, 2, 3 oder 4 C-Atomen oder Perfluoralkyl mit 1 oder 2 C-Atomen;
oder
R(61) und R(62)
gemeinsam 4 oder 5 Methylengruppen,
von denen eine CH₂-Gruppe durch Sauerstoff, S, NH, N-CH₃ oder N-Benzyl ersetzt sein kann;
oder
U einen N-haltigen Heterocyclus mit 1, 2, 3, 4, 5, 6, 7, 8 oder 9 C-Atomen,
wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(63)R(64);
wobei jedoch mindestens einer der Substituenten R5, R6, R7 und R8 ungleich Wasserstoff ist;
sowie deren pharmazeutisch verträgliche Salze.

Besonders bevorzugt sind Verbindungen der Formel I, in denen bedeuten:
mindestens einer der Substituenten R1, R3, R4, R5, R6, R7 und R8
XYₐ WZ oder X'Yₐ WZ';
X O;
Y Alkylen mit 1, 2, 3, 4 oder 5 CH₂-Gruppen,
wobei eine dieser CH₂-Gruppen durch o-, p- oder m-Phenylen ersetzt sein kann;
a Null oder 1;
W CH₂ oder - falls W nicht unmittelbar auf ein Heteroatom der Gruppe XYₐ folgt - auch O oder NR(14);
R(14) H oder Methyl;
Z C(=O)R(15) oder - falls W nicht O oder NR(14) bedeutet - auch NR(16)R(17);
R(15) N=C(NH₂)₂, NR(18)R(19) oder OR(20);
R(18) und R(19)
unabhängig voneinander H oder Alkyl mit 1, 2, 3 oder 4 C-Atomen;
oder
R(18) und R(19)
gemeinsam 4 oder 5 Methylengruppen,
von denen eine CH₂-Gruppe durch Sauerstoff, NH, N-CH₃ oder N-Benzyl ersetzt sein kann;
R(20) H oder Alkyl mit 1, 2 oder 3 C-Atomen;
R(16) und R(17)
unabhängig voneinander H oder Alkyl mit 1, 2, 3 oder 4 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
X' C(=O)NR(30), C(=O)O oder SO₂NR(30),
wobei die Verknüpfung mit dem Naphthalinring jeweils über das links stehende Atom erfolgt;
R(30) H oder Methyl;
Z' C(=O)R(15), einen N-haltigen Heterocyclus mit 1, 2, 3, 4, 5, 6, 7, 8 oder 9 C-Atomen,
wobei der N-haltige Heterocyclus über N oder C verknüpft ist;
R(15) N=C(NH₂)₂, NR(18)R(19) oder OR(20);
R(18) und R(19)
unabhängig voneinander H oder Alkyl mit 1, 2, 3 oder 4 C-Atomen;
oder
R(18) und R(19)
gemeinsam 4 oder 5 Methylengruppen,
von denen eine CH₂-Gruppe durch Sauerstoff, NH, N-CH₃ oder N-Benzyl ersetzt sein kann;
R(20) H oder Alkyl mit 1, 2 oder 3 C-Atomen;
oder
Z' - falls W nicht O oder NR(14) bedeutet - NR(16)R(17); R(16) und R(17)
unabhängig voneinander H oder Alkyl mit 1, 2, 3 oder 4 C-Atomen;
oder
R(16) und R(17)
gemeinsam 4 oder 5 Methylengruppen,
von denen eine CH₂-Gruppe durch Sauerstoff, S, NH, N-CH₃ oder N-Benzyl ersetzt sein kann;
und die jeweils übrigen Substituenten R1, R3, R4, R5, R6, R7 und R8, die noch nicht durch die zuvor gegebenen Definitionen vergeben sind,
unabhängig voneinander H, F, Cl, Br, I, CF₃ oder VₚQ_{q}U;
V O, SO₂, NR(60), OC=O, C(=O)NR(60), C(=O)O oder CR(66)R(67);
R(60), R(66) und R(67)
unabhängig voneinander H oder Alkyl mit 1, 2 oder 3 C-Atomen;
p Null oder 1;
q Null;
U H, Alkyl mit 1, 2 oder 3 C-Atomen, C(=O)R(65), oder Phenyl,
das nicht substituiert oder substituiert ist mit 1 - 3 Substituenten ausgewählt aus der Gruppe bestehend aus F, Cl, Br, CF₃, Methyl, Methoxy und NR(63)R(64); R(63) und R(64)
unabhängig voneinander H oder Methyl;
R(65) N=C(NH₂)₂;
oder
U einen N-haltigen Heterocyclus mit 1, 2, 3, 4, 5, 6, 7, 8 oder 9 C-Atomen,
wobei der N-haltige Heterocyclus über N oder C verknüpft ist;
wobei jedoch mindestens einer der Substituenten R5, R6, R7 und R8 ungleich Wasserstoff ist;
sowie deren pharmazeutisch verträgliche Salze.

Enthalten die Verbindungen der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste und Perfluoralkylreste können sowohl geradkettig als auch verzweigt vorliegen.

Als N-haltige Heterocyclen mit 1, 2, 3, 4, 5, 6, 7, 8 oder 9 C-Atomen gelten insbesondere Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Isoindolyl, Benzimidazolyl, Indazolyl, Chinolyl, Isochinolyl, Phthalazinyl, Chinoxalinyl, Chinazolinyl, Cinnolinyl.

Besonders bevorzugt sind die N-haltigen Heterocyclen Pyrrolyl, Imidazolyl, Chinolyl, Pyrazolyl, Pyridyl, Pyrazinyl, Pyrimidinyl und Pyridazinyl.

Die Erfindung betrifft weiterhin ein Verfahren zur Herstellung der Verbindung I, dadurch gekennzeichnet, daß man eine Verbindung der Formel II 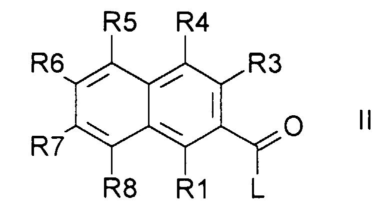 worin L für eine leicht nucleophil substituierbare leaving group steht, und die übrigen Substituenten die zuvor genannte Bedeutung haben, mit Guanidin umsetzt.
Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Naphthoesäurederivaten (Formel II, L = OH) herstellen, wie beispielsweise die Methylester der Formel II mit L = OCH₃ durch Behandeln mit gasförmigem HCl in Methanol, die Imidazolide der Formel II durch Behandeln mit Carbonyldiimidazol [L = 1-Imidazolyl, Staab, Angew. Chem. Int. Ed. Engl. 1, 351 - 367 (1962)], die gemischten Anhydride II mit Cl-COOC₂H₅ oder Tosylchlorid in Gegenwart von Triethylamin in einem inerten Lösungsmittel; die Aktivierung der Carbonsäuren kann auch mit Dicyclohexylcarbodiimid (DCC) oder mit O-[(Cyano(ethoxycarbonyl)methylen)amino]-1,1,3,3-tetramethyluroniumtetrafluoborat ("TOTU") [Proceedings of the 21. European Peptide Symposium, Peptides 1990, Editors E. Giralt and D. Andreu, Escom, Leiden, 1991] erfolgen. Eine Reihe geeigneter Methoden zur Herstellung von aktivierten Carbonsäurederivaten der Formel II ist unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Naphthoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan oder DMF gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigem Guanidin zur Abbindung der Halogenwasserstoffsäure.

Ein Teil der zugrundeliegenden Naphthoesäurederivate der Formel II ist bekannt und in der Literatur beschrieben. Die unbekannten Verbindungen der Formel II können nach literaturbekannten Methoden hergestellt werden.

Carbonsäureguanidine I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Lactate, Sulfate, Citrate, Tartrate, Acetate, Phosphate, Methylsulfonate, p-Toluolsulfonate, Maleinate, Fumarate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 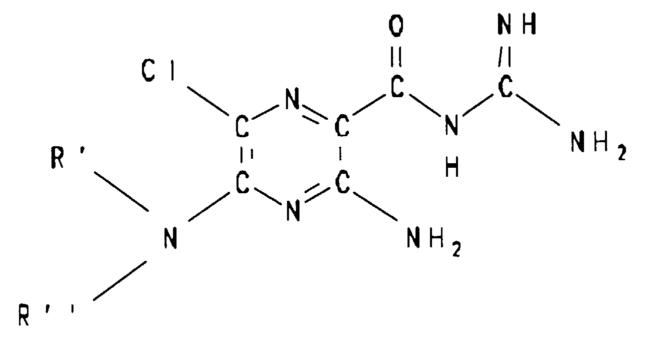 Amilorid: R' , R" = H
Dimethylamilorid: R' ,R" = CH₃
Ethylisopropylamilorid: R' = C₂H₅, R" = CH(CH₃)₂

Darüber hinaus sind Untersuchungen bekannt geworden, die auf antiarrhythmische Eigenschaften von Amilorid hinweisen [Circulation 79, 1257 - 63 (1989)]. Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (suppl.1): 167 (1988) (book of abstracts)]. So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

Aus dem US-Patent 4 251 545 sind 1-Naphthoylguanidine bekannt, die in 4-Stellung mit langkettigen Alkoxygruppen substituiert sind. Für diese Verbindungen ist eine Anwendung als Fungizide im Pflanzenschutz beschrieben. In der Offenlegungsschrift WO 94/26709 werden 2-Naphthoylguanidine beansprucht, die in den den Resten R5, R6, R7 und R8 entsprechenden Positionen Wasserstoffatome tragen.

Die EP-OS 682 017 (Hoe 94/F 123) beschreibt neben bicyclischen Heteroaroylguanidinen auch einige Naphthoylguanidine, wobei die Substituenten R1 bis R8 jedoch andere Bedeutungen als in der vorliegenden Anmeldung haben.

Als einziges Beispiel ist in diesen Patentveröffentlichungen WO 94/26709 und EP-OS 682 017 (Hoe 94/F 123) das unsubstituierte 2-Naphthoylguanidin aufgeführt.

Die aus den genannten Publikationen bekannten Verbindungen erfüllen jedoch nicht alle Anforderungen, die für die Entwicklung eines Medikamentes aus einer pharmakologisch wirksamen Verbindung erforderlich sind. So wären z.B. bessere Resorption, günstigere Halbwertszeiten, bessere Wasserlöslichkeit, geringere Toxizität oder höhere Selektivität wünschenswert.
Dies ist durch die erfindungsgemäßen Verbindungen gelungen, die außerdem keine unerwünschten und nachteiligen salidiuretischen, jedoch sehr gute antiarrhythmische Eigenschaften aufweisen, wie sie beispielsweise für die Behandlung von Krankheiten wichtig sind, die durch Sauerstoffmangel bewirkt werden. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺ /H⁺ -Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Es wurde außerdem gefunden, daß Verbindungen der Formel I eine günstige Beeinflussung der Serumlipoproteine zeigen. Es ist allgemein anerkannt, daß für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken.. Es wurde nun gefunden, daß Verbindungen der Formel I bezüglich der Beeinflussung der Serumlipidspiegel wertvolle therapeutisch verwertbare Eigenschaften zeigen. So erniedrigen sie signifikant die erhöhten Serum Konzentration von LDL und VLDL, wie sie beispielweise durch erhöhte dietätische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen die Verbindungen der Formel I zu einer deutlichen Reduktion der durch Stoffwechselanomalien induzierten Infarkte und insbesondere zu einer signifikanten Verminderung der induzierten Infarktgröße und dessen Schweregrades. Weiterhin führen Verbindungen der Formel I zu einem wirksamen Schutz gegen durch Stoffwechselanomalien induzierte Endothelschädigungen. Mit diesem Schutz der Gefäße gegen das Syndrom der endothelialen Dysfunktion sind Verbindungen der Formel I wertvolle Arzneimittel zur Prävention und zur Behandlung koronarer Gefäßsspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten; die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter cardialer Hypertrophien und Cardiomyopathien, zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten, eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, erweist sich als günstige Kombination mit verstärkter Wirkung und vermindertem Wirkstoffeinsatz.

Beansprucht wird deshalb auch die Gabe von Natrium-Protonen-Austausch-Hemmern der Formel I als Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit dbes zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Experimenteller Teil

### Liste der Abkürzungen:

- CDI: Carbonyldiimidazol
- DMF: N,N-Dimethylformamid
- RT: Raumtemperatur
- Fp: Schmelzpunkt
- FC: Flash-Chromatographie
- THF: Tetrahydrofuran
- eq.: Äquivalent
- EE: Ethylacetat (EtOAc)

### Allgemeine Vorschriften zur Herstellung von Naphthoylguanidinen (I)

### Variante 1 A: aus Naphthoesäuren (II, L = OH)

1.0 eq. des Naphthoesäurederivates der Formel II löst bzw. suspendiert man in wasserfreiem THF ( 5 ml/mmol ) und versetzt sodann mit 1.2 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck ( im Rotationsverdampfer) ab, versetzt mit Wasser und filtriert das entsprechende Guanidid (Formel I) ab. Die so erhaltenen Carbonsäureguanidine können durch Behandeln mit wäßriger, methanolischer oder etherischer Salzsäure oder anderen pharmakologisch verträglichen Säuren in die entsprechenden Salze übergeführt werden.

### Variante 1 B: aus Naphthoesäure-alkylestern (II, L = O-Alkyl)

1.0 eq. des Carbonsäure-alkylesters der Formel II sowie 5.0 eq. Guanidin (freie Base) werden in Isopropanol oder in THF gelöst oder suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotationsverdampfer) abdestilliert, der Rückstand wird in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1: 6-(2-Diethylaminoethoxy)-2-naphthoylguanidin-dihydrochlorid

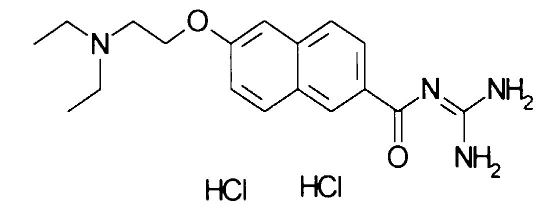

a) 6-Hydroxy-2-naphthoesäure wird in wasserfreiem Methanol, das zuvor mit HCl-Gas gesättigt wurde, 2 h unter Rückfluß erhitzt. Man engt ein, kristallisiert den Rückstand je 1 x aus Methanol und aus Aceton um und erhält 6-Hydroxy-2-naphthoesäuremethylester.
b) 3.2 g 6-Hydroxy-2-naphthoesäuremethylester und 1.28 g Natriummethylat werden in 50 ml DMF 20 min bei 40°C unter N₂ gerührt. Dann fügt man 3.65 g Diethylaminoethylchlorid zu und rührt noch 2 h bei 40°C nach. Das DMF wird am Rotationsverdampfer abgezogen, der Rückstand in verdünnter Salzsäure aufgenommen und mit Essigester extrahiert. Die wäßrige Phase wird mit Natronlauge alkalisch gestellt und erneut mit Essigester extrahiert. Nach Einengen dieses Extraktes erhält man 2.7 g 6-(2-Diethylaminoethoxy)-2-naphthoesäuremethylester.
c) 3.1 g 6-(2-Diethylaminoethoxy)-2-naphthoesäuremethylester und 1.2 g KOH werden in 20 ml Methanol gelöst und 3 h unter Rückfluß erhitzt. Nach Abziehen des Methanols wird der Rückstand in 20 ml Wasser gelöst und mit 10-prozentiger Salzsäure auf pH 2.5 gestellt. Der ausgefallene Niederschlag wird abgesaugt, und man erhält 2,7 g 6-(2-Diethylaminoethoxy)-2-naphthoesäure.
d) Zu einer Suspension von 1.0 g 6-(2-Diethylaminoethoxy)-2-naphthoesäure in 20 ml DMF werden 0.79 g CDI zugegeben, und die Reaktionsmischung wird über Nacht bei RT gerührt. Dann versetzt man mit 1.0 g Guanidin, rührt erneut über Nacht, engt ein und verrührt den Rückstand mit 20 ml Wasser. Der gebildete Niederschlag wird abgesaugt, in Ether suspendiert und nach Zugabe von etherischer Salzsäure noch 3 h gerührt. Nach Absaugen erhält man 0.9 g 6-(2-Diethylaminoethoxy)-2-naphthoylguanidin-dihydrochlorid;
   Fp.: 225°C.
   ¹H-NMR (DMSO-d6): δ [ppm] = 1.3 (6H), 3.25 (4H), 3.6 (2H), 4.55 (2H), 7.4 (1H), 7.55 (1H), 8.05 (2H), 8.15 (1H), 8.65 (2H), 8.9 (2H), 8.95 (1H), 10.5 (1H), 12.3 (1H).

### Beispiel 2: 6-(2-Diisopropylaminoethoxy)-2-naphthoylguanidin-dihydrochlorid

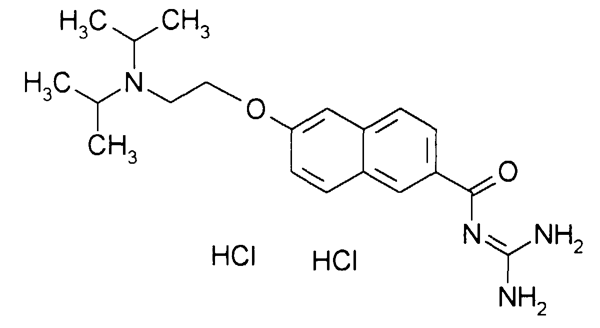

a) Aus 3.2 g 6-Hydroxy-2-naphthoesäuremethylester und 4.4 g Diisopropylaminoethylchlorid (erhalten aus dem Hydrochlorid mit Natronlauge und Extraktion mit Ether) werden analog Beispiel 1 b 4.2 g 6-(2-Diisopropylaminoethoxy)-2-naphthoesäuremethylester erhalten.
b) 3.6 g 6-(2-Diisopropylaminoethoxy)-2-naphthoesäuremethylester und 1.8 g KOH werden in Methanol 5 h unter Rückfluß erhitzt. Nach Einengen und Aufnehmen in Wasser, wird mit Salzsäure auf pH 6 gestellt, wobei das Produkt ausfällt. Man erhält 3.3 g 6-(2-Diisopropylaminoethoxy)-2-naphthoesäure; Fp.: 183-184°C.
c) 3.3 g 6-(2-Diisopropylaminoethoxy)-2-naphthoesäure und 3.1 g CDI werden in THF suspendiert und über Nacht gerührt. Nach Zugabe von 3.2 g Guanidin wird weitere 4 h bei RT gerührt, das Lösungsmittel weitgehend abgezogen und der Rückstand auf 170 ml Wasser gegossen. Das ausgefallene Produkt wird abgesaugt, in 150 ml Methanol und 50 ml Essigester gelöst und mit überschüssiger etherischer Salzsäure versetzt. Man erhält 1.8 g 6-(2-Diisopropylaminoethoxy)-2-naphthoylguanidin-dihydrochlorid;
   Fp.: 259-260°C.
   ¹H-NMR (DMSO-d6): δ [ppm] = 1.5 (12H), 3.7 (2H), 3.9 (2H), 4.7 (2H), 7.5 (1H), 7.6 (1H), 8.1 (2H), 8.3 (1H), 8.8 (2H), 9.0 (2H), 9.1 (1H), 9.9 (1H), 12.5 (1H).

### Beispiel 3: 6-[2-(4-Morpholinyl)ethoxy]-2-naphthoylguanidin-dihydrochlorid

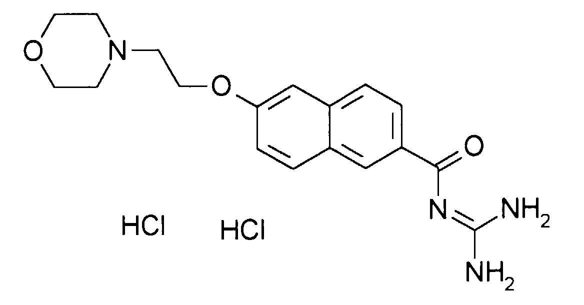

a) 6.4 g 6-Hydroxy-2-naphthoesäuremethylester und 2.56 g Natriummethylat werden in 100 ml DMF 30 min bei 40°C unter N₂ gerührt. Dann fügt man 8.04 g N-(2-Chlorethyl)-morpholin zu und rührt 2 h bei 80°C. Die Reaktionsmischung wird auf Wasser gegossen, und der ausgefallene Niederschlag wird abgesaugt. Man erhält 9.2 g 6-[2-(4-Morpholinyl)ethoxy]-2-naphthoesäuremethylester.
b) Durch Verseifung des 6-[2-(4-Morpholinyl)ethoxy]-2-naphthoesäuremethylesters analog Beispiel 1 c und anschließende Umsetzung mit CDI und Guanidin gemäß der allgemeinen Vorschrift, Variante 1 a, erhält man 6-[2-(4-Morpholinyl)ethoxy]-2-naphthoylguanidin-dihydrochlorid;
   Fp. 254-256°C.

### Beispiel 4: 6-(Guanidinocarbonylmethoxy)-2-naphthoylguanidin-dihydrochlorid

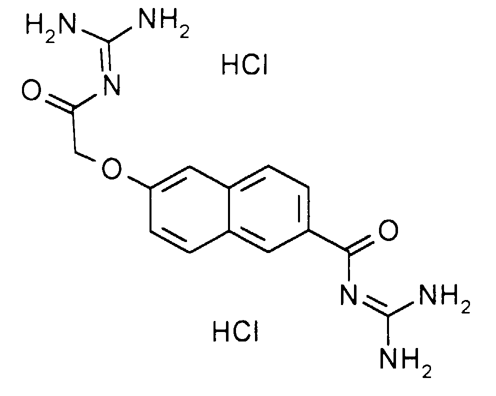

a) 3.2 g 6-Hydroxy-2-naphthoesäuremethylester und 1.28 g Natriummethylat werden in 50 ml DMF 15 min bei 40°C unter N₂ gerührt. Dann fügt man 4.1 g Bromessigsäuremethylester zu und rührt 6 h bei 70°C. Die Reaktionsmischung wird auf 100 ml Wasser gegossen, und der ausgefallene Niederschlag wird abgesaugt und aus iso-Propanol umkristallisiert. Man erhält 2.3 g 6-(Methoxycarbonylmethoxy)-2-naphthoesäuremethylester;
   Fp. 138-139°C.
b) 2.3 g 6-(Methoxycarbonylmethoxy)-2-naphthoesäuremethylester und 2.3 g KOH werden in 100 ml Wasser und 10 ml DMF 3 h unter Rückfluß erhitzt. Dann wird mit Salzsäure auf pH 0.3 angesäuert, und der ausgefallene Niederschlag wird bei RT abgesaugt. Man erhält 1.7 g 6-(Carboxymethoxy)-2-naphthoesäuremethylester;
   Fp. > 285°C.
c) 1.7 g 6-(Carboxymethoxy)-2-naphthoesäuremethylester und 3.1 g CDI werden in 50 ml DMF gelöst und über Nacht bei RT gerührt. Nach Zugabe von 2.85 g Guanidin wird erneut über Nacht bei RT gerührt, das Lösungsmittel wird im Vakuum abdestilliert und der Rückstand mit Wasser verrührt. Der gebildete Niederschlag wird abgesaugt, in Methanol gelöst und mit Essigester/HCI versetzt. Man erhält 1.5 g 6-(Guanidinocarbonylmethoxy)-2-naphthoylguanidindihydrochlorid;
   Fp.: 264-267°C.
   ¹H-NMR (DMSO-d6): δ [ppm] = 5.0 (2H), 7.5 - 8.1 (5H), 8.4 - 8.8 (8H), 8.9 (1H), 11.7 (1H), 12.2 (1H).

### Beispiel 5: 6-(3-Pyridylmethylaminocarbonyl)-2-naphthoylguanidin-dihydrochlorid

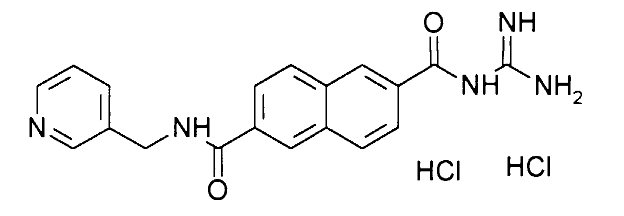

a) 4.9 g Naphthalin-2,6-dicarbonsäuredimethylester und 0.7 g KOH (85%ig) werden in 150 ml Methylenchlorid und 30 ml Methanol gelöst und über Nacht bei Raumtemperatur gerührt. Das ausgefallene Kaliumsalz wird abgesaugt, in Wasser gelöst und mit Salzsäure angesäuert. Nach Absaugen des Niederschlages erhält man 2.55 g Naphthalin-2,6-dicarbonsäuremonomethylester.
b) 2.07 g Naphthalin-2,6-dicarbonsäuremonomethylester und 1.62 g CDI werden in 25 ml DMF über Nacht bei RT gerührt. Dann fügt man 1.08 g 3-Picolylamin hinzu, rührt 3 h nach, engt ein und verrührt den Rückstand mit Wasser. Man saugt das ausgefallene Produkt ab und erhält 1.35 g 6-Methoxycarbonyl-naphthalin-2-carbonsäure-N-(3-pyridylmethyl)-amid.
c) 1.2 g 6-Methoxycarbonyl-naphthalin-2-carbonsäure-N-(3-pyridylmethyl)-amid und 0.5 g KOH werden in 25 ml Methanol suspendiert und 4 Tage bei RT gerührt. Man engt am Rotationsverdampfer ein, verrührt mit 20 ml Methylenchlorid, saugt den Niederschlag ab und löst ihn in 20 ml Wasser. Nach Ansäuern auf pH 6.2 fällt das Produkt aus, und man erhält 0.8 g 6-Carboxynaphthalin-2-carbonsäure-N-(3-pyridylmethyl)-amid; Fp > 250°C.
d) 0.75 g 6-Carboxy-naphthalin-2-carbonsäure-N-(3-pyridylmethyl)-amid werden analog Beispiel 1 d mit CDI und Guanidin umgesetzt. Man erhält 0.5 g 6-(3-Pyridylmethylaminocarbonyl)-2-naphthoylguanidin-dihydrochlorid; Fp.: 285°C.
   ¹H-NMR (DMSO-d6): δ [ppm] = 4.75 (2H), 7.95 (2H), 8.15 (4H), 8.45 (1H), 8.55-9.0 (7H), 9.65 (1H), 12.4 (1H).

### Beispiel 6: 6-[2-(Guanidinocarbonyloxy)-ethylaminocarbonyl]-2-naphthoylguanidin-dihydrochlorid

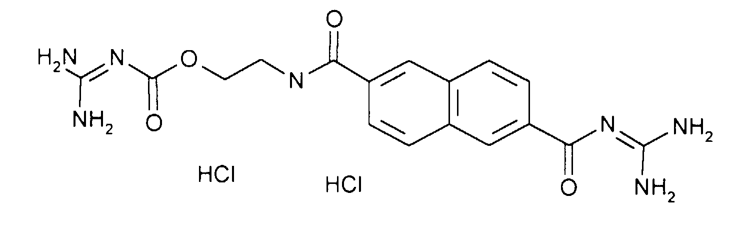

a) 2.1 g Naphthalin-2,6-dicarbonsäuremonomethylester und 25 ml Ethanolamin werden 3 h auf 150°C erhitzt. Das überschüssige Ethanolamin wird im Vakuum abdestilliert, und der Rückstand wird in Wasser gelöst und mit Essigester extrahiert. Nach Ansäuern der wäßrigen Phase wird das ausgefallene Produkt abgesaugt und aus Ethanol umkristallisiert. Man erhält 1.2 g 6-(2-Hydroxyethylaminocarbonyl)-2-naphthoesäure.
b) 1.2 g 6-(2-Hydroxyethylaminocarbonyl)-2-naphthoesäure und 1.9 g CDI werden in 13 ml DMF gelöst und über Nacht bei RT gerührt. Man fügt 2.2 g Guanidin hinzu, rührt erneut über Nacht, engt ein, versetzt mit Wasser und filtriert den ausgefallenen Niederschlag ab. Man erhält 0.94 g 6-[2-(Guanidinocarbonyloxy)-ethylaminocarbonyl]-2-naphthoylguanidin; Fp. 205°C. Durch Auflösen in 2 Moläquivalenten verdünnter Salzsäure und anschließende Gefriertrocknung erhält man das entsprechende Dihydrochlorid; Fp. ca. 270°C.

### Beispiel 7: 6-{2-[4-(4-Chlorphenyl)-1-piperazinyl]-ethoxy}-2-naphthoylguanidin-dihydrochlorid

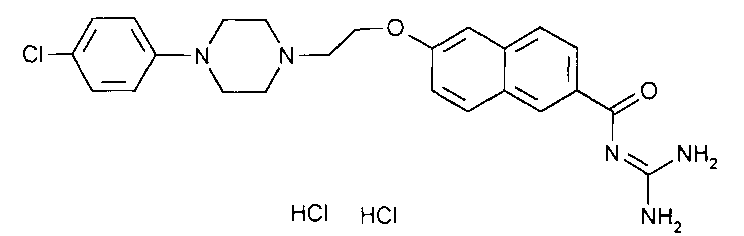

a) 2.11 g 6-Hydroxy-2-naphthoesäuremethylester und 0.84 g Natriummethylat werden in 50 ml DMF 30 min bei 40°C unter N₂ gerührt. Dann fügt man 4.6 g 4-(p-Chlorphenyl)-piperazinoethylchlorid zu und rührt 8 h bei 40°C. Die Reaktionsmischung wird mit 350 ml Methanol verdünnt und der ausgefallene Niederschlag wird abgesaugt. Man löst in 80 ml DMF und 10 ml Wasser, versetzt mit 3.8 g KOH und rührt 10 h bei 100°C. Das DMF wird am Rotationsverdampfer weitgehend abgezogen, der Rückstand wird auf Wasser gegossen, angesäuert und der ausgefallene Niederschlag wird abgesaugt. Man erhält 2.5 g 6-{2-[4-(4-Chlorphenyl)-1-piperazinyl]-ethoxy}-2-naphthoesäure; Fp. 197 - 199°C.
b) 2.4 g 6-{2-[4-(4-Chlorphenyl)-1-piperazinyl]-ethoxy}-2-naphthoesäure werden nach der allgemeinen Vorschrift (Variante 1 a) mit CDI und Guanidin umgesetzt. Man erhält 1.4 g 6-{2-[4-(4-Chlorphenyl)-1-piperazinyl]-ethoxy}-2-naphthoylguanidin-dihydrochlorid; Fp.: 269-271°C.
   ¹H-NMR (DMSO-d6): δ [ppm] = 3.3 (4H), 3.7 (4H), 3.9 (2H), 4.7 (2H), 7.0 (2H), 7.3 (2H), 7.4 (1H), 7.6 (1H), 8.0 (2H), 8.2 (1H), 8.6 (2H), 8.0 (3H), 11.4 (1H), 12.3 (1H).

### Beispiel 8 : 4-Diethylaminoethyloxy-6-methoxy-1-methyl-2-naphthoylguanidindihydrochlorid

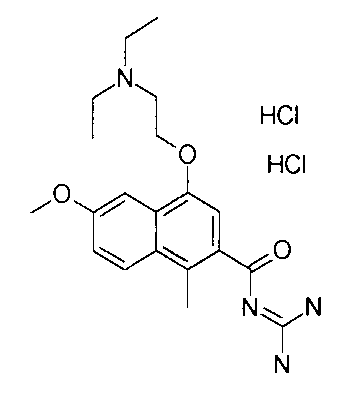

a) Zu einer Lösung von 11.2 g (0.1 mol) Kalium-tert.-butanolat in 100 ml tert. Butanol wird bei 70°C eine Mischung von 15 g (0.1 mol) p-Methoxyacetophenon und 29 g (0.2 mol) Bernsteinsäuredimethylester gelöst in 100 ml tert.-Butanol zugetropft. Man erhitzt noch 4 h auf 70°C, engt dann die Reaktionsmischung ein, versetzt mit Wasser und extrahiert zweimal mit Essigester. Nach Ansäuern der wäßrigen Phase, wird das ausgeschiedene Produkt mit Essigester extrahiert und der Extrakt über MgSO₄ getrocknet und im Vakuum eingeengt. Der Rückstand (21 g) wird mit 6.2 g (0.08 mol) Natriumacetat in 120 ml Acetanhydrid 4 h unter Rückfluß erhitzt. Man engt den Ansatz im Vakuum ein, versetzt mit Wasser, extrahiert mit Essigester und wäscht mit Sodalösung. Nach Einengen der organischen Phase und Reinigung durch Mitteldruckchromatographie erhält man 8.0 g 4-Acetoxy-6-methoxy-1-methyl-2-naphthoesäuremethylester.
b) 7.5 g (26 mmol) 4-Acetoxy-6-methoxy-1-methyl-2-naphthoesäuremethylester in 200 ml Methanol werden mit 9.4 g (52 mmol) 30-prozentiger Natriummethanolatlösung 30 min bei Raumtemperatur gerührt. Man engt ein, säuert mit verdünnter Salzsäure an und extrahiert mit Essigester. Nach Trocknen der organischen Phase über MgSO₄ und Einengen im Vakuum erhält man 4.1 g 4-Hydroxy-6-methoxy-1-methyl-2-naphthoesäuremethylester.
c) 1.5 g (6.1 mmol) 4-Hydroxy-6-methoxy-1-methyl-2-naphthoesäuremethylester und 0.49 g (9.1 mmol) Natriummethylat werden in 25 ml DMF 15 min bei 40°C unter Argon gerührt. Dann fügt man 1.4 g Diethylaminoethylchlorid zu und rührt 30 min bei 40°C nach. Das DMF wird am Rotationsverdampfer abgezogen, der Rückstand wird in verdünnter Salzsäure aufgenommen, und es wird mit Essigester extrahiert. Die wäßrige Phase wird mit Natronlauge alkalisch gestellt und erneut mit Essigester extrahiert. Nach Einengen dieses Extraktes erhält man 1.8 g rohen 4-Diethylaminoethyloxy-6-methoxy-1-methyl-2-naphthoesäuremethylester, der durch 2-stündiges Kochen mit 1 g KOH in 20 ml Methanol direkt zur Säure verseift wird. Nach dem Einengen der Reaktionsmischung wird der Rückstand in 20 ml Wasser gelöst und mit konzentrierter Salzsäure auf pH 5.5 angesäuert, wobei das Produkt ausfällt. Man erhält 0.5 g 4-Diethylaminoethyloxy-6-methoxy-1-methyl-2-naphthoesäure; Fp. 170°C.
d) Zu einer Suspension von 0.5 g 4-Diethylaminoethyloxy-6-methoxy-1-methyl-2-naphthoesäure in 18 ml THF werden 0.35 g CDI zugegeben, und die erhaltene Lösung wird 4 h bei RT gerührt. Dann fügt man 0.44 g Guanidin hinzu, rührt 1.5 h nach, engt ein und verrührt den Rückstand mit Wasser. Das ausgefallene Produkt wird abgesaugt, und man erhält 0.23 g 4-Diethylaminoethyloxy-6-methoxy-1-methyl-2-naphthoylguanidin, Fp. 135°C, das mit etherischer Salzsäure in das Dihydrochlorid, Fp. 235°C, überführt werden kann.

### Pharmakologische Daten:

### Inhibition des Na⁺/H⁺-Exchangers von Kaninchenerythrozyten:

Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrozyten via Na⁺/H⁺-Austausch flammenphotometrisch bestimmen zu können. Das Blut wurde den Ohrarterien entnommen und durch 25 IE/ml Kalium-Heparin ungerinnbar gemacht. Ein Teil jeder Probe wurde zur Doppelbestimmung des Hämatokrits durch Zentrifugation benutzt. Aliquots von jeweils 100 µl dienten zur Messung des Na⁺-Ausgangsgehalts der Erythrozyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCl, 3 KCl, 150 Sucrose, 0,1 Ouabain, 20 Tris-hydroxymethylaminomethan) bei pH 7,4 und 37°C inkubiert. Die Erythrozyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺ -Nettoinflux wurde aus der Differenz zwischen Natrium-Ausgangswerten und dem Natriumgehalt der Erythrozyten nach Inkubation errechnet. Der Amilorid-hemmbare Natrium-Influx ergab sich aus der Differenz des Natriumgehalts der Erythrozyten nach Inkubation mit und ohne Amilorid 3 x 10⁻⁴ mol/l. Auf diese Weise wurde auch bei den erfindungsgemäßen Verbindungen verfahren.

## Patentansprüche

1. Substituierte 2-Naphthoylguanidine der Formel I 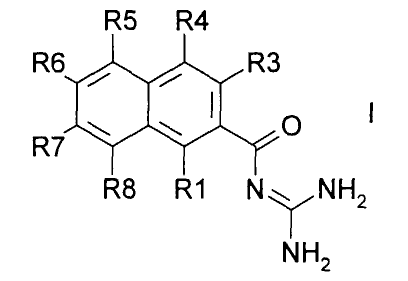 worin bedeuten:
mindestens einer der Substituenten R1, R3, R4, R5, R6, R7 und R8
XYₐ WZ oder X'YₐWZ';
X O, S, NR(10) oder CR(11)R(12);
R(10), R(11) und R(12)
unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
Y Alkylen mit 1, 2, 3, 4, 5, 6, 7 oder 8 CH₂-Gruppen,
wobei eine dieser CH₂-Gruppen durch O, S, NR(13) oder o-, p- oder m-Phenylen ersetzt sein kann;
R(13) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;
a Null oder 1;
W CH₂ oder SO₂, oder - falls W nicht unmittelbar auf ein Heteroatom der Gruppe XYₐ folgt - auch O oder NR(14);
R(14) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;
Z C(=O)R(15), SO₂R(15) oder - falls W nicht O oder NR(14) bedeutet - auch NR(16)R(17);
R(15)
N=C(NH₂)₂, NR(18)R(19), N(CH₂)_{b}NR(18)R(19) oder OR(20);
b 2 oder 3;
R(18) und R(19)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(18) und R(19)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
R(20)
H, Alkyl mit 1, 2, 3, 4, 5 oder 6 C-Atomen,
Perfluoralkyl mit 1, 2, 3 oder 4 C-Atomen oder
Cycloalkyl mit 3, 4, 5, 6 oder 7 C-Atomen;
R(16) und R(17)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
X' C=O, C(=O)NR(30), C(=O)O, SO, SO₂, SO₂NR(30), OC=O, NR(30)C=0 oder NR(30)SO₂,
wobei die Verknüpfung mit dem Naphthalinring jeweils über das links stehende Atom erfolgt;
R(30) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
Z' C(=O)R(15), SO₂R(15), einen N-haltigen Heterocyclus mit 1, 2, 3, 4, 5, 6, 7, 8 oder 9 C-Atomen,
wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(21)R(22);
R(21) und R(22)
unabhängig voneinander H, Alkyl mit 1, 2, 3 oder 4 C-Atomen oder Perfluoralkyl mit 1, 2, 3 oder 4 C-Atomen;
R(15)
N=C(NH₂)₂, NR(18)R(19), N(CH₂)_{b}NR(18)R(19) oder OR(20);
R(18) und R(19)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(18) und R(19)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
b 2 oder 3;
R(20) H, Alkyl mit 1, 2, 3, 4, 5 oder 6 C-Atomen,
Perfluoralkyl mit 1, 2, 3 oder 4 C-Atomen oder
Cycloalkyl mit 3, 4, 5, 6 oder 7 C-Atomen;
oder
Z' - falls W nicht O oder NR(14) bedeutet - NR(16)R(17);
R(16) und R(17)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
und die jeweils übrigen Substituenten R1, R3, R4, R5, R6, R7 und R8, die noch nicht durch die zuvor gegebenen Definitionen vergeben sind,
unabhängig voneinander H, F, Cl, Br, I, CN, NO₂, CF₃, C₂F₅ oder VₚQ_{q}U;
V O, S, SO, SO₂, NR(60), OC=O, C=O, C(=O)NR(60), C(=O)O oder CR(66)R(67);
R(60), R(66) und R(67)
unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
p Null oder 1;
Q Alkylen mit 1, 2, 3, 4, 5, 6, 7 oder 8 CH₂-Gruppen,
wobei eine dieser CH₂-Gruppen durch O, S, NR(68) oder o-, p- oder m-Phenylen ersetzt sein kann;
R(68)
H, Alkyl mit 1, 2, 3, 4, 5 oder 6 C-Atomen,
Perfluoralkyl mit 1, 2, 3 oder 4 C-Atomen oder
Cycloalkyl mit 3, 4, 5 oder 6 C-Atomen;
q Null oder 1;
U H, Alkyl mit 1, 2, 3, 4, 5, 6 oder 7 C-Atomen, Cycloalkyl mit 3, 4, 5, 6 oder 7 C-Atomen, C(=O)R(65), SO₂R(65), NR(61)R(62) oder Phenyl,
das nicht substituiert oder substituiert ist mit 1 - 3 Substituenten ausgewählt aus der Gruppe bestehend aus F, Cl, Br, CF₃, Methyl, Methoxy und NR(63)R(64);
R(63) und R(64)
unabhängig voneinander H, Alkyl mit 1, 2, 3 oder 4 C-Atomen oder Perfluoralkyl mit 1, 2, 3 oder 4 C-Atomen;
R(65) N=C(NH₂)₂, NR(61)R(62) oder OR(60);
R(61) und R(62)
unabhängig voneinander H, Alkyl mit 1, 2, 3, 4, 5, 6, 7 oder 8 C-Atomen oder Perfluoralkyl mit 1, 2, 3 oder 4 C-Atomen;
oder
R(61) und R(62)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
oder
U einen N-haltigen Heterocyclus mit 1, 2, 3, 4, 5, 6, 7, 8 oder 9 C-Atomen,
wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(63)R(64);
wobei jedoch mindestens einer der Substituenten R5, R6, R7 und R8 ungleich Wasserstoff ist;
sowie deren pharmazeutisch verträgliche Salze.

2. Verbindung der Formel I nach Anspruch 1, in der bedeuten: mindestens einer der Substituenten R1, R3, R4, R5, R6, R7 und R8
XYₐ WZ oder X'YₐWZ';
X O, S, NR(10);
R(10) H, Alkyl mit 1, 2, 3 oder 4 C-Atomen, Perfluoralkyl mit 1 oder 2 C-Atomen oder Cycloalkyl mit 3, 4, 5 oder 6 C-Atomen;
Y Alkylen mit 1, 2, 3, 4 oder 5 CH₂-Gruppen,
wobei eine dieser CH₂-Gruppen durch O, S, NR(13) oder o-, p- oder m-Phenylen ersetzt sein kann;
R(13) H, Alkyl mit 1, 2, 3 oder 4 C-Atomen oder Cycloalkyl mit 3, 4, 5 oder 6 C-Atomen;
a Null oder 1;
W CH₂ oder - falls W nicht unmittelbar auf ein Heteroatom der Gruppe XYₐ folgt - auch O oder NR(14);
R(14) H, Alkyl mit 1, 2, 3 oder 4 C-Atomen;
Z C(=O)R(15), SO₂R(15) oder- falls W nicht O oder NR(14) bedeutet - auch NR(16)R(17);
R(15) N=C(NH₂)₂, NR(18)R(19), N(CH₂)_{b}NR(18)R(19) oder
OR(20);
b 2 oder 3;
R(18) und R(19)
unabhängig voneinander H oder Alkyl mit 1, 2, 3, 4 oder 5 C-Atomen;
oder
R(18) und R(19)
gemeinsam 4 oder 5 Methylengruppen,
von denen eine CH₂-Gruppe durch Sauerstoff, S, NH, N-CH₃ oder N-Benzyl ersetzt sein kann;
R(20) H, Alkyl mit 1, 2, 3, 4, 5 oder 6 C-Atomen;
R(16) und R(17)
unabhängig voneinander H, Alkyl mit 1, 2, 3, 4 oder 5 C-Atomen oder Perfluoralkyl mit 1 oder 2 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
X' C(=O)NR(30), C(=O)O, SO₂NR(30), OC=O, NR(30)C=O oder NR(30)SO₂,
wobei die Verknüpfung mit dem Naphthalinring jeweils über das links stehende Atom erfolgt;
R(30) H, Alkyl mit 1, 2, 3 oder 4 C-Atomen, Perfluoralkyl mit 1 oder 2 C-Atomen oder Cycloalkyl mit 3, 4, 5 oder 6 C-Atomen;
Z' C(=O)R(15), SO₂R(15), einen N-haltigen Heterocyclus mit 1, 2, 3, 4, 5, 6, 7, 8 oder 9 C-Atomen,
wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(21)R(22);
R(21) und R(22)
unabhängig voneinander H oder Alkyl mit 1, 2, 3 oder 4 C-Atomen;
R(15) N=C(NH₂)₂, NR(18)R(19), N(CH₂)_{b}NR(18)R(19) oder OR(20);
R(18) und R(19)
unabhängig voneinander H, Alkyl mit 1, 2, 3, 4 oder 5 C-Atomen;
oder
R(18) und R(19)
gemeinsam 4 oder 5 Methylengruppen,
von denen eine CH₂-Gruppe durch Sauerstoff, S, NH, N-CH₃ oder N-Benzyl ersetzt sein kann;
b 2 oder 3;
R(20) H, Alkyl mit 1, 2, 3, 4, 5 oder 6 C-Atomen;
oder
Z' - falls W nicht O oder NR(14) bedeutet - NR(16)R(17);
R(16) und R(17)
unabhängig voneinander H, Alkyl mit 1, 2, 3, 4 oder 5 C-Atomen oder Perfluoralkyl mit 1 oder 2 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann; und die jeweils übrigen Substituenten R1, R3, R4, R5, R6, R7 und R8, die noch nicht durch die zuvor gegebenen Definitionen vergeben sind,
unabhängig voneinander H, F, Cl, Br, I, CN, NO₂, CF₃, C₂F₅ oder VₚQ_{q}U;
V O, S, SO₂, NR(60), OC=O, C=O, C(=O)NR(60), C(=O)O oder CR(66)R(67);
R(60), R(66) und R(67)
unabhängig voneinander H, Alkyl mit 1, 2 oder 3 C-Atomen, Perfluoralkyl mit 1 oder 2 C-Atomen oder Cycloalkyl mit 3, 4, 5 oder 6 C-Atomen;
p Null oder 1;
q Null;
U H, Alkyl mit 1, 2, 3 oder 4 C-Atomen, Cycloalkyl mit 3, 4, 5 oder 6 C-Atomen, C(=O)R(65), NR(61)R(62) oder Phenyl,
das nicht substituiert oder substituiert ist mit 1 - 3 Substituenten ausgewählt aus der Gruppe bestehend aus F, Cl, Br, CF₃, Methyl, Methoxy und NR(63)R(64);
R(63) und R(64)
unabhängig voneinander H, Alkyl mit 1, 2, 3 oder 4 C-Atomen;
R(65) N=C(NH₂)₂ oder OR(60);
R(61) und R(62)
unabhängig voneinander H, Alkyl mit 1, 2, 3 oder 4 C-Atomen oder Perfluoralkyl mit 1 oder 2 C-Atomen;
oder
R(61) und R(62)
gemeinsam 4 oder 5 Methylengruppen,
von denen eine CH₂-Gruppe durch Sauerstoff, S, NH, N-CH₃ oder N-Benzyl ersetzt sein kann;
oder
U einen N-haltigen Heterocyclus mit 1, 2, 3, 4, 5, 6, 7, 8 oder 9 C-Atomen,
wobei der N-haltige Heterocyclus über N oder C verknüpft ist und nicht substituiert oder substituiert ist mit 1 - 3 Substituenten ausgewählt aus der Gruppe bestehend aus F, Cl, Br, CF₃, Methyl, Methoxy und NR(63)R(64);
wobei jedoch mindestens einer der Substituenten R5, R6, R7 und R8 ungleich Wasserstoff ist.

3. Verbindung der Formel I nach Anspruch 1 oder 2, in der bedeuten: mindestens einer der Substituenten R1, R3, R4, R5, R6, R7 und R8
XYₐ WZ oder X'Yₐ WZ';
X O;
Y Alkylen mit 1, 2, 3, 4 oder 5 CH₂-Gruppen,
wobei eine dieser CH₂-Gruppen durch o-, p- oder m-Phenylen ersetzt sein kann;
a Null oder 1;
W CH₂ oder - falls W nicht unmittelbar auf ein Heteroatom der Gruppe XYₐ folgt - auch O oder NR(14);
R(14) H oder Methyl;
Z C(=O)R(15) oder - falls W nicht O oder NR(14) bedeutet - auch NR(16)R(17);
R(15) N=C(NH₂)₂, NR(18)R(19) oder OR(20);
R(18) und R(19)
unabhängig voneinander H oder Alkyl mit 1, 2, 3 oder 4 C-Atomen;
oder
R(18) und R(19)
gemeinsam 4 oder 5 Methylengruppen,
von denen eine CH₂-Gruppe durch Sauerstoff, NH, N-CH₃ oder N-Benzyl ersetzt sein kann;
R(20) H oder Alkyl mit 1, 2 oder 3 C-Atomen;
R(16) und R(17)
unabhängig voneinander H oder Alkyl mit 1, 2, 3 oder 4 C-Atomen;
oder
R(16) und R(17)
gemeinsam 4 oder 5 Methylengruppen,
von denen eine CH₂-Gruppe durch Sauerstoff, S, NH, N-CH₃, N-Benzyl oder N-(p-Chlorphenyl) ersetzt sein kann;
X' C(=O)NR(30), C(=O)O oder SO₂NR(30),
wobei die Verknüpfung mit dem Naphthalinring jeweils über das links stehende Atom erfolgt;
R(30) H oder Methyl;
Z' C(=O)R(15), einen N-haltigen Heterocyclus mit 1, 2, 3, 4, 5, 6, 7, 8 oder 9 C-Atomen,
wobei der N-haltige Heterocyclus über N oder C verknüpft ist;
R(15) N=C(NH₂)₂, NR(18)R(19) oder OR(20);
R(18) und R(19)
unabhängig voneinander H oder Alkyl mit 1, 2, 3 oder 4 C-Atomen;
oder
R(18) und R(19)
gemeinsam 4 oder 5 Methylengruppen,
von denen eine CH₂-Gruppe durch Sauerstoff, NH, N-CH₃ oder N-Benzyl ersetzt sein kann;
R(20) H oder Alkyl mit 1, 2 oder 3 C-Atomen;
oder
Z' - falls W nicht O oder NR(14) bedeutet - NR(16)R(17);
R(16) und R(17)
unabhängig voneinander H oder Alkyl mit 1, 2, 3 oder 4 C-Atomen;
oder
R(16) und R(17)
gemeinsam 4 oder 5 Methylengruppen,
von denen eine CH₂-Gruppe durch Sauerstoff, S, NH, N-CH₃ oder N-Benzyl ersetzt sein kann;
und die jeweils übrigen Substituenten R1, R3, R4, R5, R6, R7 und R8, die noch nicht durch die zuvor gegebenen Definitionen vergeben sind,
unabhängig voneinander H, F, Cl, Br, I, CF₃ oder VₚQ_{q}U;
V O, SO₂, NR(60), OC=O, C(=O)NR(60), C(=O)O oder CR(66)R(67);
R(60), R(66) und R(67)
unabhängig voneinander H oder Alkyl mit 1, 2 oder 3 C-Atomen;
p Null oder 1;
q Null;
U H, Alkyl mit 1, 2 oder 3 C-Atomen, C(=O)R(65) oder Phenyl, das nicht substituiert oder substituiert ist mit 1 - 3 Substituenten ausgewählt aus der Gruppe bestehend aus F, Cl, Br, CF₃, Methyl, Methoxy und NR(63)R(64);
R(63) und R(64)
unabhängig voneinander H oder Methyl;
R(65) N=C(NH₂)₂;
oder
U einen N-haltigen Heterocyclus mit 1, 2, 3, 4, 5, 6, 7, 8 oder 9 C-Atomen,
wobei der N-haltige Heterocyclus über N oder C verknüpft ist;
wobei jedoch mindestens einer der Substituenten R5, R6, R7 und R8 ungleich Wasserstoff ist.

4. Verfahren zur Herstellung einer Verbindung I nach Anspruch 1, dadurch gekennzeichnet daß man eine Verbindung der Formel II 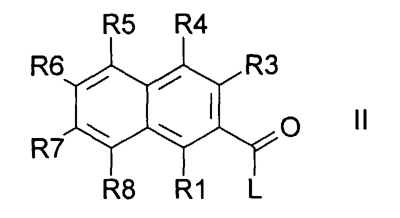 worin L für eine leicht nucleophil substituierbare leaving group steht, und die übrigen Substituenten wie in Anspruch 1 definiert sind,
mit Guanidin umsetzt.

5. Verwendung einer Verbindung I nach Anspruch 1 zur Herstellung eines Medikaments zur Behandlung von Arrhythmien.

6. Verwendung einer Verbindung I nach Anspruch 1 zur Herstellung eines Medikaments zur Behandlung oder Prophylaxe des Herzinfarkts.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung I nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch I zur Herstellung eines Medikaments zur Behandlung von Krankheiten, bei denen die Zellproliferation eine primäre oder sekundäre Ursache darstellt.

15. Verwendung einer Verbindung I nach Anspruch 1 zur Herstellung eines Medikaments zur Behandlung oder Prophylaxe von Störungen des Fettstoffwechsels.

16. Arzneimittel, dadurch gekennzeichnet, dass es eine Verbindung der Formel I nach einem oder mehreren der Ansprüche 1 bis 3 enthält.

## Claims

1. A substituted 2-naphthoylguanidine of the formula I 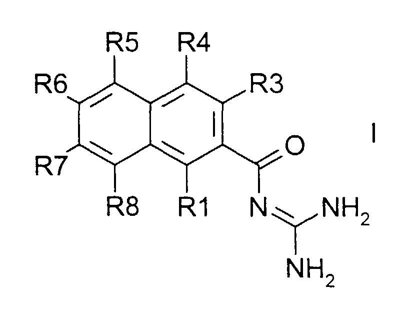 in which:
at least one of the substituents R1, R3, R4, R5, R6, R7 and R8 is
XYₐWZ or X'YₐWZ';
X is O, S, NR(10) or CR(11)R(12);
R(10), R(11) and R(12)
independently of one another are H, alkyl having 1, 2, 3, 4, 5 or 6 carbon atoms, perfluoroalkyl having 1, 2, 3 or 4 carbon atoms or cycloalkyl having 3, 4, 5, 6 or 7 carbon atoms;
Y is alkylene having 1, 2, 3, 4, 5, 6, 7 or 8 CH₂ groups,
where one of these CH₂ groups can be replaced by O, S, NR(13) or o-, p- or m-phenylene;
R(13) is H, alkyl having 1, 2, 3, 4, 5 or 6 carbon atoms, perfluoroalkyl having 1, 2, 3 or 4 carbon atoms or cycloalkyl having 3, 4, 5 or 6 carbon atoms;
a is zero or 1;
W is CH₂ or SO₂, or - if W does not immediately follow a heteroatom of the group XYₐ - alternatively O or NR(14);
R(14) is H, alkyl having 1, 2, 3, 4, 5 or
6 carbon atoms, perfluoroalkyl having 1, 2, 3 or 4 carbon atoms or cycloalkyl having 3, 4, 5 or 6 carbon atoms;
Z is C(=O)R(15), SO₂R(15) or - if W is not O or NR(14) - alternatively NR(16)R(17);
R(15) is
N=C(NH₂)₂, NR(18)R(19), N(CH₂)_{b}NR(18)R(19) or OR(20);
b is 2 or 3;
R(18) and R(19)
independently of one another are H, alkyl having 1, 2, 3, 4, 5, 6, 7 or 8 carbon atoms or perfluoroalkyl having 1, 2, 3 or 4 carbon atoms;
or
R(18) and R(19)
together are 4 or 5 methylene groups,
of which one CH₂ group can be replaced by oxygen, S, NH, N-CH₃, N-benzyl or N-(p-chlorophenyl);
R(20) is
H, alkyl having 1, 2, 3, 4, 5 or 6 carbon atoms, perfluoroalkyl having 1, 2, 3 or 4 carbon atoms or cycloalkyl having 3, 4, 5, 6 or 7 carbon atoms;
R(16) and R(17)
independently of one another are H, alkyl having 1, 2, 3, 4, 5, 6, 7 or 8 carbon atoms or perfluoroalkyl having 1, 2, 3 or 4 carbon atoms;
or,
R(16) and R(17)
together are 4 or 5 methylene groups,
of which one CH₂ group can be replaced by oxygen, S, NH, N-CH₃, N-benzyl or N-(p-chlorophenyl);
X' is C=O, C(=O)NR(30), C(=O)O, SO, SO₂, SO₂NR(30), OC=O, NR(30)C=O or NR(30)SO₂,
where the linkage with the naphthalene ring in each case takes place via the left atom;
R(30) is H, alkyl having 1, 2, 3, 4, 5 or
6 carbon atoms, perfluoroalkyl having 1, 2, 3 or
4 carbon atoms or cycloalkyl having 3, 4, 5, 6 or
7 carbon atoms;
Z' is C(=O)R(15), SO₂R(15), an N-containing heterocycle having 1, 2, 3, 4, 5, 6, 7, 8 or 9 carbon atoms,
where the N-containing heterocycle is linked via N or C and is unsubstituted or substituted by 1 - 3 substituents selected from the group consisting of F, Cl, Br, CF₃, methyl, methoxy and NR(21)R(22);
R(21) and R(22)
independently of one another are H, alkyl having 1, 2, 3 or 4 carbon atoms or perfluoroalkyl having 1, 2, 3 or 4 carbon atoms;
R(15) is
N=C(NH₂)₂, NR(18)R(19), N(CH₂)_{b}NR(18)R(19) or OR(20);
R(18) and R(19)
independently of one another are H, alkyl having 1, 2, 3, 4, 5, 6, 7 or 8 carbon atoms or perfluoroalkyl having 1, 2, 3 or 4 carbon atoms;
or
R(18) and R(19)
together are 4 or 5 methylene groups,
of which one CH₂ group can be replaced by oxygen, S, NH, N-CH₃, N-benzyl or N-(p-chlorophenyl);
b is 2 or 3;
R(20) is H, alkyl having 1, 2, 3, 4,
5 or 6 carbon atoms, perfluoroalkyl having 1, 2, 3 or 4 carbon atoms or cycloalkyl having 3, 4, 5, 6 or 7 carbon atoms;
or
Z' - if W is not O or NR(14) - is NR(16) R(17);
R(16) and R(17)
independently of one another are H, alkyl having 1, 2, 3, 4, 5, 6, 7 or 8 carbon atoms or perfluoroalkyl having 1, 2, 3 or 4 carbon atoms;
or
R(16) and R(17)
together are 4 or 5 methylene groups,
of which one CH₂ group can be replaced by oxygen, S, NH, N-CH₃, N-benzyl or N-(p-chlorophenyl);
and the other substituents R1, R3, R4, R5, R6, R7 and R8 in each case, which are still not allocated by the definitions given above,
independently of one another are H, F, Cl, Br, I, CN, NO₂, CF₃, C₂F₅ or V_{P}Q_{q}U;
V is O, S, SO, SO₂, NR(60), OC=O, C=O, C(=O)NR(60), C(=O)O or CR(66)R(67);
R(60), R(66) and R(67)
independently of one another are H, alkyl having 1, 2, 3, 4, 5 or 6 carbon atoms, perfluoroalkyl having 1, 2, 3 or 4 carbon atoms or cycloalkyl having 3, 4, 5, 6 or 7 carbon atoms;
p is zero or 1;
Q is alkylene having 1, 2, 3, 4, 5, 6, 7 or 8 CH₂ groups,
where one of these CH₂ groups can be replaced by O, S, NR(68) or o-, p- or m-phenylene;
R(68) is
H, alkyl having 1, 2, 3, 4, 5 or 6 carbon atoms, perfluoroalkyl having 1, 2, 3 or 4 carbon atoms or cycloalkyl having 3, 4, 5 or 6 carbon atoms;
q is zero or 1;
U is H, alkyl having 1, 2, 3, 4, 5, 6 or 7 carbon atoms, cycloalkyl having 3, 4, 5, 6 or 7 carbon atoms, C(=O)R(65), SO₂R(65), NR(61)R(62) or phenyl,
which is unsubstituted or substituted by 1 - 3 substituents selected from the group consisting of F, Cl, Br, CF₃, methyl, methoxy and
NR(63)R(64);
R(63) and R(64)
independently of one another are H, alkyl having 1, 2, 3 or 4 carbon atoms or perfluoroalkyl having 1, 2, 3 or 4 carbon atoms;
R(65) is N=C(NH₂)₂, NR(61)R(62) or OR(60);
R(61) and R(62)
independently of one another are H, alkyl having 1, 2, 3, 4, 5, 6, 7 or 8 carbon atoms or perfluoroalkyl having 1, 2, 3 or 4 carbon atoms;
or
R(61) and R(62)
together are 4 or 5 methylene groups,
of which one CH₂ group can be replaced by oxygen, S, NH, N-CH₃, N-benzyl or N-(p-chlorophenyl);
or
U is an N-containing heterocycle having 1, 2, 3, 4, 5, 6, 7, 8 or 9 carbon atoms,
where the N-containing heterocycle is linked via N or C and is unsubstituted or substituted by 1 - 3 substituents selected from the group consisting of F, Cl, Br, CF₃, methyl, methoxy and NR(63)R(64);
but where at least one of the substituents R5, R6, R7 and R8 is not equal to hydrogen;
or its pharmaceutically tolerable salts.

2. A compound of the formula I as claimed in claim 1, in which: at least one of the substituents R1, R3, R4, R5, R6, R7 and R8 is
XYₐWZ or X'YₐWZ';
X is O, S, NR(10);
R(10) is H, alkyl having 1, 2, 3 or 4
carbon atoms, perfluoroalkyl having I or 2 carbon atoms or cycloalkyl having 3, 4, 5 or 6 carbon atoms;
Y is alkylene having 1, 2, 3, 4 or 5 CH₂ groups,
where one of these CH₂ groups can be replaced by O, S, NR(13) or o-, p- or m-phenylene;
R(13) is H, alkyl having 1, 2, 3 or 4
carbon atoms or cycloalkyl having 3, 4, 5 or 6 carbon atoms;
a is zero or 1;
W is CH₂ or - if W does not immediately follow a heteroatom of the group XYₐ - alternatively O or
NR(14);
R(14) is H, alkyl having 1, 2, 3 or 4 carbon atoms;
Z is C(=O)R(15), SO₂R(15) or - if W is not O or NR(14) - alternatively NR(16)R(17);
R(15) is N=C(NH₂)₂, NR(18)R(19),
N(CH₂)_{b}NR(18)R(19) or OR(20);
b is 2 or 3;
R(18) and R(19)
independently of one another are H or alkyl having 1, 2, 3, 4 or 5 carbon atoms;
or
R(18) and R(19)
together are 4 or 5 methylene groups,
of which one CH₂ group can be replaced by oxygen, S, NH, N-CH₃ or N-benzyl;
R(20) is H, alkyl having 1, 2, 3, 4, 5 or 6 carbon atoms;
R(16) and R(17)
independently of one another are H, alkyl having 1, 2, 3, 4 or 5 carbon atoms or perfluoroalkyl having 1 or 2 carbon atoms;
or
R(16) and R(17)
together are 4 or 5 methylene groups,
of which one CH₂ group can be replaced by oxygen, S, NH, N-CH₃, N-benzyl or N-(p-chlorophenyl);
X' is C(=O)NR(30), C(=O)O, SO₂NR(30), OC=O,
NR(30)C=O or NR(30)SO₂,
where the linkage with the naphthalene ring in each case takes place via the left atom;
R(30) is H, alkyl having 1, 2, 3 or 4
carbon atoms, perfluoroalkyl having I or 2 carbon atoms or cycloalkyl having 3, 4, 5 or 6 carbon atoms;
Z' is C(=O)R(15), SO₂R(15), an N-containing heterocycle having 1, 2, 3, 4, 5, 6, 7, 8 or 9 carbon atoms,
where the N-containing heterocycle is linked via N or C and is unsubstituted or substituted by 1 - 3 substituents selected from the group consisting of F, Cl, Br, CF₃, methyl, methoxy and NR(21)R(22);
R(21) and R(22)
independently of one another are H or alkyl having 1, 2, 3 or 4 carbon atoms;
R(15) is N=C(NH₂)₂, NR(18)R(19),
N(CH₂)_{b}NR(18)R(19) or OR(20);
R(18) and R(19)
independently of one another are H, alkyl having 1, 2, 3, 4 or 5 carbon atoms;
or
R(18) and R(19)
together are 4 or 5 methylene groups,
of which one CH₂ group can be replaced by oxygen, S, NH, N-CH₃ or N-benzyl;
b is 2 or 3;
R(20) is H, alkyl having 1, 2, 3, 4,
5 or 6 carbon atoms;
or
Z' - if W is not O or NR(14) - is NR(16)R(17);
R(16) and R(17)
independently of one another are H, alkyl having 1, 2, 3, 4 or 5 carbon atoms or perfluoroalkyl having I or 2 carbon atoms;
or
R(16) and R(17)
together are 4 or 5 methylene groups, of which one CH₂ group can be replaced by oxygen, S, NH, N-CH₃, N-benzyl or N-(p-chlorophenyl);
and the other substituents R1, R3, R4, R5, R6, R7 and R8 in each case, which are still not allocated by the definitions given above,
independently of one another are H, F, Cl, Br, I, CN, NO₂, CF₃, C₂F₅ or V_{P}Q_{q}U;
V is O, S, SO₂, NR(60), OC=O, C=O, C(=O)NR(60),
C(=O)O or CR(66)R(67);
R(60), R(66) and R(67)
independently of one another are H, alkyl having 1, 2 or 3 carbon atoms, perfluoroalkyl having I or 2 carbon atoms or cycloalkyl having 3, 4, 5 or 6 carbon atoms;
p is zero or 1;
q is zero;
U is H, alkyl having 1, 2, 3 or 4 carbon atoms, cycloalkyl having 3, 4, 5 or 6 carbon atoms, C(=O)R(65),
NR(61)R(62) or phenyl,
which is unsubstituted or substituted by 1 - 3 substituents selected from the group consisting of F, Cl, Br, CF₃, methyl, methoxy and
NR(63)R(64);
R(63) and R(64)
independently of one another are H, alkyl having 1, 2, 3 or 4 carbon atoms;
R(65) is N=C(NH₂)₂ or OR(60);
R(61) and R(62)
independently of one another are H, alkyl having 1, 2, 3 or 4 carbon atoms or perfluoroalkyl having 1 or 2 carbon atoms;
or
R(61) and R(62)
together are 4 or 5 methylene groups,
of which one CH₂ group can be replaced by oxygen, S, NH, N-CH₃ or N-benzyl;
or
U is an N-containing heterocycle having 1, 2, 3, 4, 5, 6, 7, 8 or 9 carbon atoms,
where the N-containing heterocycle is linked via N or C and is unsubstituted or substituted by 1 - 3 substituents selected from the group consisting of F, Cl, Br, CF₃, methyl, methoxy and NR(63)R(64);
but where at least one of the substituents R5, R6, R7 and R8 is not equal to hydrogen.

3. A compound of the formula I as claimed in claim 1 or 2, in which: at least one of the substituents R1, R3, R4, R5, R6, R7 and R8 is
XYₐWZ or X'YₐWZ';
X is O;
Y is alkylene having 1, 2, 3, 4 or 5 CH₂ groups,
where one of these CH₂ groups can be replaced by o-, p- or m-phenylene;
a is zero or 1;
W is CH₂ or - if W does not immediately follow a heteroatom of the group XYₐ - alternatively O or NR(14);
R(14) is H or methyl;
Z is C(=O)R(15), or - if W is not O or NR(14) - alternatively NR(16)R(17);
R(15) is N=C(NH₂)₂, NR(18)R(19) or OR(20);
R(18) and R(19)
independently of one another are H or alkyl having 1, 2, 3 or 4 carbon atoms;
or
R(18) and R(19)
together are 4 or 5 methylene groups,
of which one CH₂ group can be replaced by oxygen, NH, N-CH₃ or N-benzyl;
R(20) is H or alkyl having 1, 2 or 3
carbon atoms;
R(16) and R(17)
independently of one another are H or alkyl having 1, 2, 3 or 4 carbon atoms;
or
R(16) and R(17)
together are 4 or 5 methylene groups,
of which one CH₂ group can be replaced by oxygen, S, NH, N-CH₃, N-benzyl or N-(p-chlorophenyl);
X' is C(=O)NR(30), C(=O)O or SO₂NR(30),
where the linkage with the naphthalene ring in each case takes place via the left atom;
R(30) is H or methyl;
Z' is C(=O)R(15), an N-containing heterocycle having 1, 2, 3, 4, 5, 6, 7, 8 or 9 carbon atoms,
where the N-containing heterocycle is linked via N or C;
R(15) is N=C(NH₂)₂, NR(18)R(19) or OR(20);
R(18) and R(19)
independently of one another are H or alkyl having 1, 2, 3 or 4 carbon atoms;
or
R(18) and R(19)
together are 4 or 5 methylene groups,
of which one CH₂ group can be replaced by oxygen, NH, N-CH₃ or N-benzyl;
R(20) is H or alkyl having 1, 2 or 3 carbon atoms;
or
Z' - if W is not O or NR(14) - is NR(16) R(17);
R(16) and R(17)
independently of one another are H or alkyl having 1, 2, 3 or 4 carbon atoms;
or
R(16) and R(17)
together are 4 or 5 methylene groups,
of which one CH₂ group can be replaced by oxygen, S, NH, N-CH₃ or N-benzyl;
and the other substituents R1, R3, R4, R5, R6, R7 and R8 in each case, which are still not allocated by the definitions given above,
independently of one another are H, F, Cl, Br, I, CF₃ or
VₚQ_{q}U;
V is O, SO₂, NR(60), OC=O, C(=O)NR(60), C(=O)O or CR(66)R(67);
R(60), R(66) and R(67)
independently of one another are H or alkyl having 1, 2 or 3 carbon atoms;
p is zero or 1;
q is zero;
U is H, alkyl having 1, 2 or 3 carbon atoms, C(=O)R(65) or phenyl,
which is unsubstituted or substituted by 1 - 3 substituents selected from the group consisting of F, Cl, Br, CF₃, methyl, methoxy and
NR(63)R(64);
R(63) and R(64)
independently of one another are H or methyl;
R(65) is N=C(NH₂)₂;
or
U is an N-containing heterocycle having 1, 2, 3, 4, 5, 6, 7, 8 or 9 carbon atoms,
where the N-containing heterocycle is linked via N or C;
but where at least one of the substituents R5, R6, R7 and R8 is not equal to hydrogen.

4. A process for the preparation of a compound I as claimed in claim 1, which comprises reacting a compound of the formula II 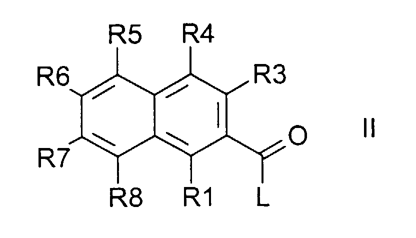 in which L is an easily nucleophilically substitutable leaving group, and the other substituents are as defined in claim 1,
with guanidine.

5. The use of a compound I as claimed in claim 1 for the production of a medicament for the treatment of arrhythmias.

6. The use of a compound I as claimed in claim I for the production of a medicament for the treatment or prophylaxis of cardiac infarct.

7. The use of a compound I as claimed in claim I for the production of a medicament for the treatment or prophylaxis of angina pectoris.

8. The use of a compound I as claimed in claim 1 for the production of a medicament for the treatment or prophylaxis of ischemic conditions of the heart.

9. The use of a compound I as claimed in claim I for the production of a medicament for the treatment or prophylaxis of ischemic conditions of the peripheral and central nervous system and of stroke.

10. The use of a compound I as claimed in claim 1 for the production of a medicament for the treatment or prophylaxis of ischemic conditions of the peripheral organs and limbs.

11. The use of a compound I as claimed in claim 1 for the production of a medicament for the treatment of states of shock.

12. The use of a compound I as claimed in claim 1 for the production of a medicament for use in surgical operations and organ transplants.

13. The use of a compound I as claimed in claim I for the production of a medicament for the preservation and storage of transplants for surgical measures.

14. The use of a compound I as claimed in claim 1 for the production of a medicament for the treatment of diseases in which cell proliferation is a primary or secondary cause.

15. The use of a compound I as claimed in claim 1 for the production of a medicament for the treatment or prophylaxis of disorders of lipid metabolism.

16. A pharmaceutical, comprising a compound of the formula I as claimed in one or more of claims I to 3.

## Revendications

1. 2-naphtoylguanidines substituées de formule I 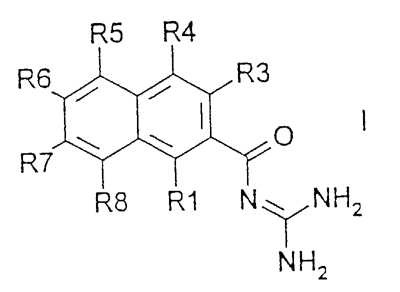 dans laquelle au moins l'un des substituants R1, R3, R4, R5, R6, R7 et R8 représente
XYₐWZ ou X'YₐWZ';
X représente O, S, NR(10) ou CR(11)R(12);
R(10), R(11) et R(12)
représentent, indépendamment les uns des autres, H ou un groupe alkyle ayant 1, 2, 3, 4, 5 ou 6 atomes de carbone, perfluoroalkyle ayant 1, 2, 3 ou 4 atomes de carbone, ou cycloalkyle ayant 3, 4, 5, 6 ou 7 atomes de carbone;
Y représente un groupe alkylène comportant 1, 2, 3, 4, 5, 6, 7 ou 8 groupes CH₂,
l'un de ces groupes CH₂ pouvant être remplacé par 0, S, NR(13) ou par un groupe o-, p- ou m-phénylène,
R(13) représentant H ou un groupe alkyle ayant
1, 2, 3, 4, 5 ou 6 atomes de carbone, perfluoroalkyle ayant 1, 2, 3 ou 4 atomes de carbone, ou cycloalkyle ayant 3, 4, 5 ou 6 atomes de carbone;
a représente zéro ou 1;
W représente CH₂, SO₂ ou - lorsque W ne fait pas immédiatement suite à un hétéroatome du groupe XYₐ - également O ou NR(14),
R(14) représentant H ou un groupe alkyle ayant 1, 2, 3, 4, 5 ou 6 atomes de carbone, perfluoroalkyle ayant 1, 2, 3 ou 4 atomes de carbone, ou cycloalkyle ayant 3, 4, 5 ou 6 atomes de carbone;
Z représente un groupe C(=O)R(15), SO₂R(15) ou - lorsque W ne représente pas O ni NR(14) - également NR(16)R(17),
R(15) représentant
N=C(NH₂)₂, NR(18)R(19), N(CH₂)_{b}NR(18)R(19 ou OR (20);
b étant 2 ou 3,
R(18) et R(19)
représentant, indépendamment l'un de l'autre, H ou un groupe alkyle ayant 1, 2, 3, 4, 5, 6 , 7 ou 8 atomes de carbone, ou perfluoroalkyle ayant 1, 2, 3 ou 4 atomes de carbone, ou
R(18) et R(19)
représentant ensemble 4 ou 5 groupes méthylène,
dont un groupe CH₂ peut être remplacé par un atome d'oxygène ou de soufre ou par un groupe NH, N-CH₃, N-benzyle ou N-(p-chlorophényle),
R(20)
représentant H ou un groupe alkyle ayant 1, 2, 3, 4, 5 ou 6 atomes de carbone, perfluoroalkyle ayant 1, 2, 3 ou 4 atomes de carbone, ou cycloalkyle ayant 3, 4, 5, 6 ou 7 atomes de carbone,
R(16) et R(17)
représentant, indépendamment l'un de l'autre, H ou un groupe alkyle ayant 1, 2, 3, 4, 5, 6, 7 ou 8 atomes de carbone, ou perfluoroalkyle ayant 1, 2, 3 ou 4 atomes de carbone,
ou
R(16) et R(17)
représentant ensemble 4 ou 5 groupes méthylène,
dont un groupe CH₂ peut être remplacé par un atome d'oxygène ou de soufre ou par un groupe NH, N-CH₃, N-benzyle ou N-(p-chlorophényle);
X' représente C=O, C(=O)NR(30), C(=O)O, SO, SO₂, SO₂NR(30), OC=O ou NR(30)C=O ou NR(30)SO₂,
la liaison avec le cycle naphtalène s'effectuant chaque fois par l'atome se trouvant à gauche;
R(30) représentant H ou un groupe alkyle ayant 1, 2, 3, 4, 5 ou 6 atomes de carbone, perfluoroalkyle ayant 1, 2, 3 ou 4 atomes de carbone ou cycloalkyle ayant 3, 4, 5, 6 ou 7 atomes de carbone;
Z' représente un groupe C(=O)R(15), SO₂R(15), un hétérocycle azoté ayant 1, 2, 3, 4, 5, 6, 7, 8 ou 9 atomes de carbone,
l'hétérocycle azoté étant relié par N ou C et n'étant pas substitué ou portant de 1 à 3 substituants choisi(s) dans l'ensemble constitué par F, Cl, Br, CF₃, les groupes méthyle, méthoxy et NR(21)R(22),
R(21) et R(22)
représentant, indépendamment l'un de l'autre, H ou un groupe alkyle ayant 1, 2, 3 ou 4 atomes de carbone, ou perfluoroalkyle ayant 1, 2, 3 ou 4 atomes de carbone;
R(15) représente N=C(NH₂)₂, NR(18)R(19),
N(CH₂)_{b}NR(18)R(19) ou OR(20);
R(18) et R(19)
représentant, indépendamment l'un de l'autre, H ou un groupe alkyle ayant 1, 2, 3, 4, 5, 6, 7 ou 8 atomes de carbone, ou perfluoroalkyle ayant 1, 2, 3 ou 4 atomes de carbone;
ou
R(18) et R(19)
représentant ensemble 4 ou 5 groupes méthylène,
dont un groupe CH₂ peut être remplacé par un atome d'oxygène ou de soufre ou par un groupe NH, N-CH₃, N-benzyle ou N-(p-chlorophényle),
b étant égal à 2 ou 3,
R(20) représentant H ou un groupe alkyle ayant 1, 2, 3, 4, 5 ou 6 atomes de carbone, perfluoroalkyle ayant 1, 2, 3 ou 4 atomes de carbone, ou cycloalkyle ayant 3, 4, 5, 6 ou 7 atomes de carbone;
ou
Z' représente - lorsque W ne représente pas O ni NR(14) - un groupe NR(16)R(17),
R(16) et R(17)
représentant, indépendamment l'un de l'autre, H ou un groupe alkyle ayant 1, 2, 3, 4, 5, 6, 7 ou 8 atomes de carbone, ou perfluoroalkyle ayant 1, 2, 3 ou 4 atomes de carbone,
ou
R(16) et R(17)
représentant ensemble 4 ou 5 groupes méthylène,
dont un groupe CH₂ peut être remplacé par un atome d'oxygène ou de soufre ou par un groupe NH, N-CH₃, N-benzyle ou N-(p-chlorophényle),
et les autres substituants respectifs R1, R3, R4, R5, R6 R7 et R8 qui ne sont pas encore donnés par les définitions précédentes
représentent, indépendamment les uns des autres, H, F, Cl, Br, I, CN, NO₂, CF₃, C₂F₅ ou VₚQ_{q}U;
V représente O, S' SO, SO₂, NR(60), OC=O, C=O, C(=O)NR(60), C(=O)O ou CR(66)R(67);
R(60), R(66) et R(67)
représentant, indépendamment les uns des autres, H ou un groupe alkyle ayant 1, 2, 3, 4, 5 ou 6 atomes de carbone, perfluoroalkyle ayant 1, 2, 3 ou 4 atones de carbone, ou cycloalkyle ayant 3, 4, 5, 6 ou 7 atomes de carbone;
p est zéro ou 1;
Q représente un groupe alkylène comportant 1, 2, 3, 4, 5, 6, 7 ou 8 groupes CH₂,
l'un de ces groupes CH₂ pouvant être remplacé par O, S, NR(68) ou par un groupe o-, p- ou m-phénylène;
R(68) représentant
H ou un groupe alkyle ayant 1, 2, 3, 4, 5 ou 6 atomes de carbone, perfluoroalkyle ayant 1, 2, 3 ou 4 atomes de carbone, ou cycloalkyle ayant 3, 4, 5 ou 6 atomes de carbone,
q est zéro ou 1;
U représente H ou un groupe alkyle ayant 1, 2, 3, 4, 5, 6 ou 7 atomes de carbone, cycloalkyle ayant 3, 4, 5, 6 ou 7 atomes de carbone, C(=O)R(65), SO₂R(65), NR(61)R(62) ou phényle,
qui n'est pas substitué ou porte de 1 à 3 substituants choisi(s) dans l'ensemble constitué par F, Cl, Br, CF₃, les groupes méthyle, méthoxy et NR(63)R(64),
R(63) et R(64)
représentant, indépendamment l'un de l'autre, H ou un groupe alkyle ayant 1, 2, 3 ou 4 atomes de carbone, ou perfluoroalkyle ayant 1, 2, 3 ou 4 atomes de carbone,
R(65) représente N=C(NH₂)₂, NR(61)R(62) ou ou OR(60),
R(61) et R(62)
représentent, indépendamment l'un de
l'autre, H ou un groupe alkyle ayant 1, 2, 3, 4, 5, 6, 7 ou 8 atomes de carbone, ou perfluoroalkyle ayant 1, 2, 3 ou 4 atomes de carbone;
ou
R(61) et R(62)
représentent ensemble 4 ou 5 groupes méthylène,
dont un groupe CH₂ peut être remplacé par un atome d'oxygène ou de soufre ou par un groupe NH, N-CH₃, N-benzyle ou N-(p-chlorophényle);
ou
U représente un hétérocycle azoté ayant 1, 2, 3, 4, 5, 6, 7, 8 ou 9 atomes de carbone,
l'hétérocycle azoté étant relié par N ou C et n'étant pas substitué ou portant de 1 à 3 substituants choisi(s) dans l'ensemble constitué par F, Cl, Br, CF₃, les groupes méthyle, méthoxy et NR(63)R(64),
mais au moins l'un des substituants R5, R6, R7 et R8 étant différent d'un atome d'hydrogène;
et leurs sels pharmaceutiquement acceptables.

2. Composé de formule I selon la revendication 1, dans laquelle
au moins l'un des substituants R1, R3, R4, R5, R6, R7 et R8 représente
XYₐWZ ou X'YₐWZ';
X représente O, S, NR(10);
R(10) représente H ou un groupe alkyle ayant 1, 2, 3 ou 4 atomes de carbone, perfluoroalkyle ayant 1 ou 2 atomes de carbone, ou cycloalkyle ayant 3, 4, 5 ou 6 atomes de carbone;
Y représente un groupe alkylène comportant 1, 2, 3, 4 ou 5 groupes CH₂,
l'un de ces groupes CH₂ pouvant être remplacé par O, S, NR(13) ou par un groupe o-, p- ou m-phénylène,
R(13) représentant H ou un groupe alkyle ayant 1, 2, 3 ou 4 atomes de carbone, ou cycloalkyle ayant 3, 4, 5 ou 6 atomes de carbone;
a représente zéro ou 1;
W représente CH₂ ou - lorsque W ne fait pas immédiatement suite à un hétéroatome du groupe XYₐ - également O ou NR(14),
R(14) représentant H ou un groupe alkyle ayant 1, 2, 3 ou 4 atomes de carbone;
Z représente un groupe C(=O)R(15), SO₂R(15) ou - lorsque W ne représente pas O ni NR(14) - également NR(16)R(17),
R(15) représentant
N=C(NH₂)₂, NR(18)R(19), N(CH₂)_{b}NR(18)R(19 ou OR(20);
b étant 2 ou 3,
R(18) et R(19)
représentant, indépendamment l'un de l'autre, H ou un groupe alkyle ayant 1, 2, 3, 4 ou 5 atomes de carbone,
ou
R(18) et R(19)
représentant ensemble 4 ou 5 groupes méthylène,
dont un groupe CH₂ peut être remplacé par un atome d'oxygène ou de soufre ou par un groupe NH, N-CH₃ ou N-benzyle,
R(20)
représentant H ou un groupe alkyle ayant 1, 2, 3, 4, 5 ou 6 atomes de carbone,
R(16) et R(17)
représentant, indépendamment l'un de l'autre, H ou un groupe alkyle ayant 1, 2, 3, 4 ou 5 atomes de carbone, ou perfluoroalkyle ayant 1 ou 2 atomes de carbone,
ou
R(16) et R(17)
représentant ensemble 4 ou 5 groupes méthylène,
dont un groupe CH₂ peut être remplacé par un atome d'oxygène ou de soufre ou par un groupe NH, N-CH₃, N-benzyle ou N-(p-chlorophényle);
X' représente C(=O)NR(30), C(=O)O, SO₂NR(30), OC=O, NR(30)C=O ou NR(30)SO₂,
la liaison avec le cycle naphtalène s'effectuant chaque fois par l'atome se trouvant à gauche;
R(30) représentant H ou un groupe alkyle ayant 1, 2, 3 ou 4 atomes de carbone, perfluoroalkyle ayant 1 ou 2 atomes de carbone ou cycloalkyle ayant 3, 4, 5 ou 6 atomes de carbone;
Z' représente un groupe C(=O)R(15), SO₂R(15), un hétérocycle azoté ayant 1, 2, 3, 4, 5, 6, 7, 8 ou 9 atomes de carbone,
l'hétérocycle azoté étant relié par N ou C et n'étant pas substitué ou portant de 1 à 3 substituants choisi(s) dans l'ensemble constitué par F, Cl, Br, CF₃, les groupes méthyle, méthoxy et NR(21)R(22),
R(21) et R(22)
représentant, indépendamment l'un de l'autre, H ou un groupe alkyle ayant 1, 2, 3 ou 4 atomes de carbone;
R(15) représente N=C(NH₂)₂, NR(18)R(19),
N(CH₂)_{b}NR(18)R(19) ou OR(20);
R(18) et R(19)
représentant, indépendamment l'un de l'autre, H ou un groupe alkyle ayant 1, 2, 3, 4 ou 5 atomes de carbone;
ou
R(18) et R(19)
représentant ensemble 4 ou 5 groupes méthylène,
dont un groupe CH₂ peut être remplacé par un atome d'oxygène ou de soufre ou par un groupe NH, N-CH₃ ou N-benzyle;
b étant égal à 2 ou 3,
R(20) représentant H ou un groupe alkyle ayant 1, 2, 3, 4, 5 ou 6 atomes de carbone;
ou
Z' représente - lorsque W ne représente pas O ni NR(14) - un groupe NR(16)R(17),
R(16) et R(17)
représentant, indépendamment l'un de l'autre, H ou un groupe alkyle ayant 1, 2, 3, 4 ou 5 atomes de carbone, ou perfluoroalkyle ayant 1 ou 2 atomes de carbone,
ou
R(16) et R(17)
représentant ensemble 4 ou 5 groupes méthylène,
dont un groupe CH₂ peut être remplacé par un atome d'oxygène ou de soufre ou par un groupe NH, N-CH₃, N-benzyle ou N-(p-chlorophényle),
et les autres substituants respectifs R1, R3, R4, R5, R6 R7 et R8 qui ne sont pas encore donnés par les définitions précédentes
représentent, indépendamment les uns des autres,
H, F, Cl, Br, I, CN, NO₂, CF₃, C₂F₅ ou VₚQ_{q}U;
V représente O, S, SO₂, NR(60), OC=O, C=O, C(=O)NR(60), C(=O)O ou CR(66)R(67);
R(60), R(66) et R(67)
représentant, indépendamment les uns des autres, H ou un groupe alkyle ayant 1, 2, ou 3 atomes de carbone, perfluoroalkyle ayant 1 ou 2 atomes de carbone, ou cycloalkyle ayant 3, 4, 5 ou 6 atomes de carbone;
p est zéro ou 1;
q est zéro;
U représente H ou un groupe alkyle ayant 1, 2, 3 ou 4 atomes de carbone, cycloalkyle ayant 3, 4, 5 ou 6 atomes de carbone, C(=O)R(65), NR(61)R(62) ou phényle,
qui n'est pas substitué ou porte de 1 à 3 substituants choisi(s) dans l'ensemble constitué par F, Cl, Br, CF₃, les groupes méthyle, méthoxy et NR(63)R(64),
R(63) et R(64)
représentant, indépendamment l'un de l'autre, H ou un groupe alkyle ayant 1, 2, 3 ou 4 atomes de carbone,
R(65) représente N=C(NH₂)₂ ou OR(60),
R(61) et R(62)
représentant, indépendamment l'un de l'autre, H ou un groupe alkyle ayant 1, 2, 3 ou 4 atomes de carbone, ou perfluoroalkyle ayant 1 ou 2 atomes de carbone;
ou
R(61) et R(62)
représentant ensemble 4 ou 5 groupes méthylène,
dont un groupe CH₂ peut être remplacé par un atome d'oxygène ou de soufre ou par un groupe NH, N-CH₃ ou N-benzyle;
ou
U représente un hétérocycle azoté ayant 1, 2, 3, 4, 5, 6, 7, 8 ou 9 atomes de carbone, l'hétérocycle azoté étant relié par N ou C et n'étant pas substitué ou portant de 1 à 3 substituants choisi(s) dans l'ensemble constitué par F, Cl, Br, CF₃, les groupes méthyle, méthoxy et NR(63)R(64),
mais au moins l'un des substituants R5, R6, R7 et R8 étant différent d'un atome d'hydrogène.

3. Composé de formule I selon la revendication 1 ou 2, dans lequel
au moins l'un des substituants R1, R3, R4, R5, R6, R7 et R8 représente
XYₐWZ ou X'YₐWZ';
X représente O;
Y représente un groupe alkylène comportant 1, 2, 3, 4 ou 5 groupes CH₂,
l'un de ces groupes CH₂ pouvant être remplacé par un groupe o-, p- ou m-phénylène,
a représente zéro ou 1;
W représente CH₂ ou - lorsque W ne fait pas immédiatement suite à un hétéroatome du groupe XYₐ - également O ou NR(14),
R(14) représentant H ou le groupe méthyle;
Z représente un groupe C(=O)R(15) ou - lorsque W ne représente pas O ni NR(14) - également NR(16)R(17),
R(15) représentant
N=C(NH₂)₂, NR(18)R(19) ou OR(20),
R(18) et R(19)
représentant, indépendamment l'un de l'autre, H ou un groupe alkyle ayant 1, 2, 3 ou 4 atomes de carbone,
ou
R(18) et R(19)
représentant ensemble 4 ou 5 groupes méthylène,
dont un groupe CH₂ peut être remplacé par un atome d'oxygène **ou par un** groupe NH, N-CH₃ ou N-benzyle,
R(20)
représentant H ou un groupe alkyle ayant 1, 2 ou 3 atomes de carbone,
R(16) et R(17)
représentant, indépendamment l'un de l'autre, H ou un groupe alkyle ayant 1, 2, 3 ou 4 atomes de carbone,
ou
R(16) et R(17)
représentant ensemble 4 ou 5 groupes méthylène,
dont un groupe CH₂ peut être remplacé par un atome d'oxygène ou de soufre ou par un groupe NH, N-CH₃, N-benzyle ou N-(p-chlorophényle);
X' représente C(=O)NR(30), C(=O)O, SO₂NR(30),
la liaison avec le cycle naphtalène s'effectuant chaque fois par l'atome se trouvant à gauche;
R(30) représentant H ou le groupe méthyle;
Z' représente un groupe C(=O)R(15), un hétérocycle azoté ayant 1, 2, 3, 4, 5, 6, 7, 8 ou 9 atomes de carbone,
l'hétérocycle azoté étant relié par N ou C; R(15) représente N=C(NH₂)₂, NR(18)R(19) ou OR(20),
R(18) et R(19)
représentant, indépendamment l'un de l'autre, H ou un groupe alkyle ayant 1, 2, 3 ou 4 atomes de carbone;
ou
R(18) et R(19)
représentant ensemble 4 ou 5 groupes méthylène,
dont un groupe CH₂ peut être remplacé par un atome d'oxygène ou par un groupe NH, N-CH₃ ou N-benzyle;
R(20) représentant H ou un groupe alkyle ayant 1, 2 ou 3 atomes de carbone;
ou
Z' représente - lorsque W ne représente pas O ni NR(14) - un groupe NR(16)R(17),
R(16) et R(17)
représentant, indépendamment l'un de l'autre, H ou un groupe alkyle ayant 1, 2, 3 ou 4 atomes de carbone,
ou
R(16) et R(17)
représentant ensemble 4 ou 5 groupes méthylène,
dont un groupe CH₂ peut être remplacé par un atome d'oxygène ou de soufre ou par un groupe NH, N-CH₃ ou N-benzyle,
et les autres substituants respectifs R1, R3, R4, R5, R6 R7 et R8 qui ne sont pas encore donnés par les définitions précédentes
représentent, indépendamment les uns des autres,
H, F, Cl, Br, I, CF₃ ou VₚQ_{q}U;
V représente O, SO₂, NR(60), OC=O, C(=O)NR(60), C(=O)O ou CR(66)R(67);
R(60), R(66) et R(67)
représentant, indépendamment les uns des autres, H ou un groupe alkyle ayant 1, 2 ou 3 atomes de carbone;
p est zéro ou 1;
q est zéro;
U représente H ou un groupe alkyle ayant 1, 2 ou 3 atomes de carbone, C(=O)R(65) ou phényle,
qui n'est pas substitué ou porte de 1 à 3 substituants choisi(s) dans l'ensemble constitué par F, Cl, Br, CF₃, les groupes méthyle, méthoxy et NR(63)R(64),
R(63) et R(64)
représentant, indépendamment l'un de l'autre, H ou le groupe méthyle,
R(65) représente N=C(NH₂)₂,
ou
U représente un hétérocycle azoté ayant 1, 2, 3, 4, 5, 6, 7, 8 ou 9 atomes de carbone,
l'hétérocycle azoté étant relié par N ou C;
mais au moins l'un des substituants R5, R6, R7 et R8 étant différent d'un atome d'hydrogène.

4. Procédé pour la préparation d'un composé I selon la revendication 1, caractérisé en ce que l'on fait réagir avec de la guanidine un composé de formule II 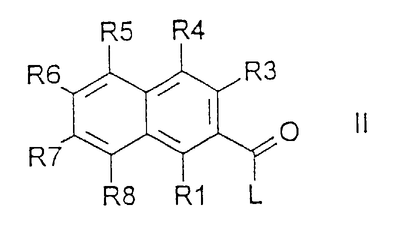 dans laquelle L représente un groupe partant pouvant être aisément remplacé par substitution nucléophile, et les autres substituants ont les significations données dans la revendication 1.

5. Utilisation d'un composé I selon la revendication 1, pour la fabrication d'un médicament destiné au traitement d'arythmies.

6. Utilisation d'un composé I selon la revendication 1, pour la fabrication d'un médicament destiné au traitement ou à la prophylaxie de l'infarctus du myocarde.

7. Utilisation d'un composé I selon la revendication 1, pour la fabrication d'un médicament destiné au traitement ou à la prophylaxie de l'angine de poitrine.

8. Utilisation d'un composé I selon la revendication 1, pour la fabrication d'un médicament destiné au traitement ou à la prophylaxie d'états ischémiques du coeur.

9. Utilisation d'un composé I selon la revendication 1, pour la fabrication d'un médicament destiné au traitement ou à la prophylaxie d'états ischémiques du système nerveux périphérique et central et de l'apoplexie cérébrale.

10. Utilisation d'un composé I selon la revendication 1, pour la fabrication d'un médicament destiné au traitement ou à la prophylaxie d'états ischémiques d'organes périphériques et des membres.

11. Utilisation d'un composé I selon la revendication 1, pour la fabrication d'un médicament destiné au traitement d'états de choc.

12. Utilisation d'un composé I selon la revendication 1, pour la fabrication d'un médicament destiné à l'utilisation dans des opérations chirurgicales et des greffes d'organes.

13. Utilisation d'un composé I selon la revendication 1, pour la fabrication d'un médicament destiné à la conservation et au stockage de transplants pour des opérations chirurgicales.

14. Utilisation d'un composé I selon la revendication 1, pour la fabrication d'un médicament destiné au traitement de maladies dans lesquelles la prolifération cellulaire représente une cause primaire ou secondaire.

15. Utilisation d'un composé I selon la revendication 1, pour la fabrication d'un médicament destiné au traitement ou à la prophylaxie de troubles du métabolisme des lipides.

16. Médicament, caractérisé en ce qu'il contient un composé de formule I selon une ou plusieurs des revendications 1 à 3.
